(19) Europäisches Patentamt
European Patent Office
Office européen des brevets

(11) Publication number: **0 171 441 A1**

(12) **EUROPEAN PATENT APPLICATION**

(21) Application number: 84109568.0

(22) Date of filing: 10.08.84

(51) Int. Cl.[4]: C 07 C 177/00
C 08 B 37/16, A 61 K 31/557

(43) Date of publication of application:
19.02.86 Bulletin 86/8

(84) Designated Contracting States:
AT BE CH DE FR GB IT LI LU NL SE

(71) Applicant: ONO PHARMACEUTICAL CO., LTD.
No. 14, Doshomachi 2-chome Higashi-ku
Osaka-shi Osaka(JP)

(72) Inventor: Arai, Yoshinobu
1-5-8-505, Wakayama-dai Shimamoto-cho
Mishima-gun Osaka(JP)

(72) Inventor: Wakatsuka, Hirohisa
3-3-708, Miyano-cho
Takatsuki-shi Osaka(JP)

(72) Inventor: Sasaki, Yutaro
11-88-504, Okayamate-cho
Hirakata-shi Osaka(JP)

(74) Representative: Bentham, Stephen et al,
J.A. Kemp & Co. 14 South Square Gray's Inn
London WC1R 5EU(GB)

(54) **Prostaglandin analogues, processes for their preparation and compositions containing them.**

(57) Prostaglandin D derivatives of the formula

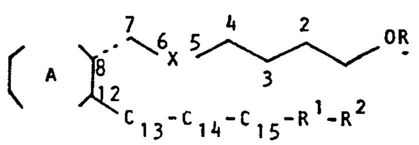
(I)

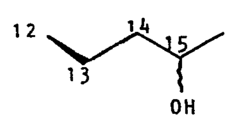
(V)

wherein
[A] represents a group of the formula:

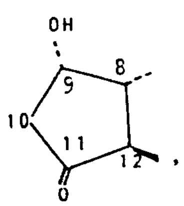
,
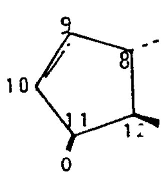
or
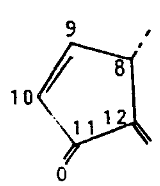

(II) (III) (IV)

X represents an ethylene group or a cis-vinylene group,
$C_{13}$-$C_{14}C_{15}$ represents

(i) a group of the formula:

./...

EP 0 171 441 A1

when

[A] represents a group of the formula (II) or (III), or (ii) a group of the formula:

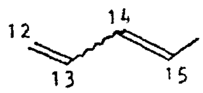

(VI)

when

[A] represents a group of the formula (IV), R represents (i) a group of the formula:

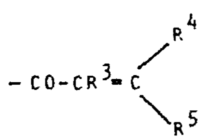

or

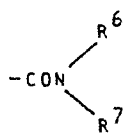

(wherein $R^3$, $R^4$, $R^5$, $R^6$ and $R^7$ are hydrogen or alkyl), when

[A] represents a group of the formula (II) or (III), or (ii) a hydrogen atom or a group of the formula:

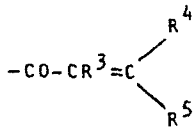

,

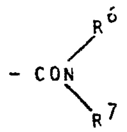

or

$-COR^8$

(wherein $R^8$ represents an alkyl group and the other symbols are as hereinbefore defined), when

[A] represents a group of the formula (IV), $R^1$ represents a single bond or alkylene, and $R^2$ represents alkyl, cycloalkyl, phenyl or phenoxy, provided that when $R^1$ represents a single bond $R^2$ does not represent phenoxy, and cyclodextrin clathrates thereof, possess anti-tumor activity and increase the number of white blood cells *in vivo*.

0171441

DESCRIPTION

PROSTAGLANDIN ANALOGUES, PROCESSES FOR THEIR PREPARATION AND COMPOSITIONS CONTAINING THEM

This invention relates to new prostaglandin D analogues, processes for their preparation and pharmaceutical compositions containing them.

Prostaglandins are derivatives of prostanoic acid having the following structure:

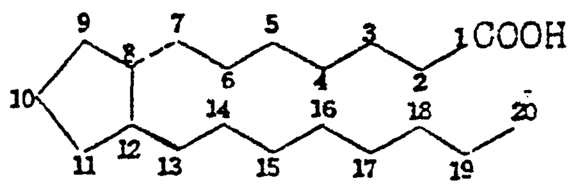

Various types of prostaglandins are known, and their types depend on the structure of the alicyclic ring and the substituents. For example, the alicyclic rings of prostaglandins F (PGF), E (PGE) and D (PGD) have the following structures, respectively:

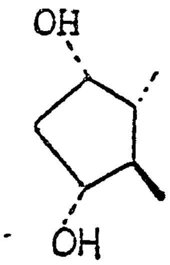

(PGF)

,

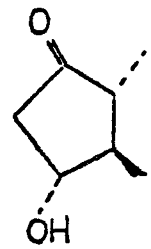

(PGE)

and

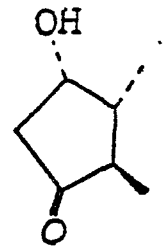

(PGD)

In the above structural formulae or in the other structural formulae in this specification, according to the generally accepted nomenclature, the dotted line indicates that the substituent attached thereto is behind the ring plane, i.e. is of the α-configuration, the bold line ◢ indicates that the substituent attached thereto is in front of the ring plane, i.e. is of the β-configuration, and the wavy line 〰 indicates that the substituent attached thereto is of the α-configuration or the β-configuration or a mixture thereof.

These compounds are sub-classified according to the positions of the double bonds in the side chains attached to the alicyclic ring at the 8-position and the 12-position. The PG-1 compound has a trans-double bond (trans-$\Delta^{13}$) between $C_{13}$-$C_{14}$ and a PG-2 compound has a cis-double bond between $C_5$-$C_6$ and a trans-double bond between $C_{13}$-$C_{14}$ (cis-$\Delta^5$, ,trans-$\Delta^{13}$). For example, prostaglandin $D_1$ ($PGD_1$) and prostaglandin $D_2$ ($PGD_2$) may be expressed by the following structural formulae, respectively:

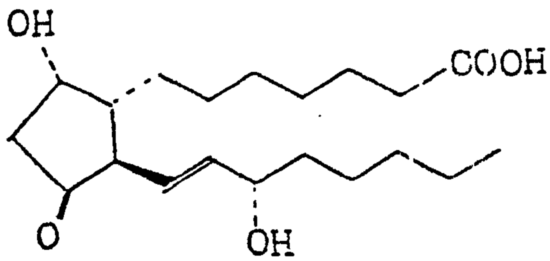

(PGD$_1$)

and

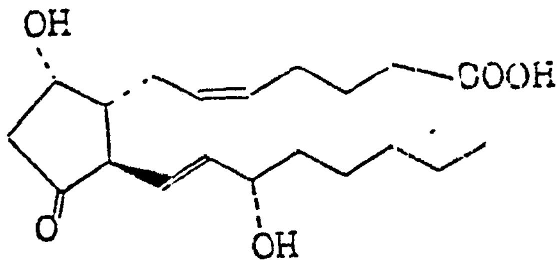

(PGD$_2$)

Further, when one or more methylene groups are removed from the aliphatic group attached at the 12-position of the alicyclic ring of a prostaglandin, said compound is known as a nor-prostaglandin according to the general rules of organic nomenclature, and the number of the removed methylene groups is indicated by adding di-, tri- , etc. before the prefix "nor".

The prostaglandins generally have pharmacological properties. For example, they exert various effects, including the stimulation of contraction of smooth muscles, a hypotensive effect, a diuretic effect, a bronchial dilation effect, the inhibition of lipolysis, the inhibition of platelet aggregation and the inhibition of gastric acid secretion. Therefore, they are useful in treatments of hypertension, thrombosis, asthma and gastric and intestinal ulcers, in the induction of labour and abortion in pregnant mammals, in the prevention of arteriosclerosis and also as diuretics. Recently, it has been found that some prostaglandins have an anti-tumour effect. The prostaglandins are liposoluble substances present in extremely small quantities in the tissues which secrete prostaglandins in vivo in animals.

As a result of research and experimentation to discover novel compounds which have the pharmacological effects of the "natural" prostaglandins, or which have one or more of these properties to an enhanced degree, or which have properties which are not found in the "natural" prostaglandins, it has been discovered that (1) certain novel PGD analogues in which the hydroxy group in the hydroxymethyl group ($-CH_2OH$)) attached to the 1-position of $PGD_1$ alcohol and $PGD_2$ alcohol analogues is replaced by an acryloyloxy or carbamoyloxy group as hereinafter defined, PGD analogues in which the hydroxy group attached at the 9-position of such compounds is dehydrated to introduce a double bond between

$C_9$-$C_{10}$, and the hydroxy group attached at the 15-position of such compounds is dehydrated to introduce double bonds between $C_{12}$-$C_{13}$ and between $C_{14}$-$C_{15}$, and (2) PGD analogues in which the hydroxy group attached at the 9-position and at the 15-position of $PGD_1$ alcohol and $PGD_2$ alcohol analogues, are dehydrated to introduce double bonds between $C_9$-$C_{10}$, $C_{12}$-$C_{13}$ and $C_{14}$-$C_{15}$, and further (3) PGD analogues in which the hydroxy group in the hydroxymethyl group attached to the 1-position of PGD analogues mentioned above in (2) is replaced by an acyloxy group as hereinafter defined, have a strong anti-tumour effect and have the effect of increasing the number of white blood cells _in vivo_.

Published Japanese Patent Application 89648/77, 89649/77 and 89650/77 described, in general terms, $PGD_1$ and $PGD_2$ alcohol derivatives, but no preparation of such a derivative was described: the Japanese specifications describe only the known pharmacological properties of known prostaglandin compunds and contain no disclosure of any anti-tumour effect. The anti-tumour effect of the compounds of the present invention has not hitherto been found in known PGD alcohol derivatives.

The PGD derivatives of the present invention, including the alcohol derivatives, in which double bonds are present between $C_9$-$C_{10}$, $C_{12}$-$C_{13}$ and $C_{14}$-$C_{15}$ are novel compounds, unexpected from conventional preparative techniques; the acryloyloxy and carbamoyloxy derivatives of

the invention are also novel compounds, unexpected from conventional preparative techniques.

The present invention accordingly provides PGD analogues of the general formula:

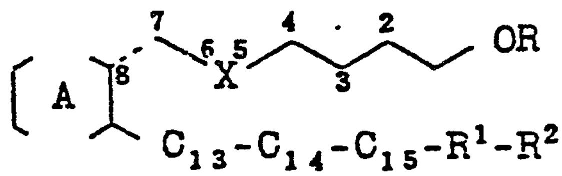

(I)

[wherein [A] represents a group of the formula:

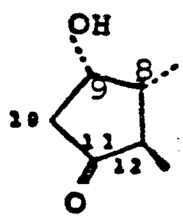

(II)

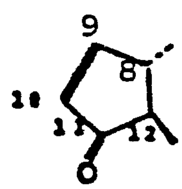

(III)

or

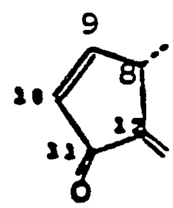

(IV)

X represents an ethylene group ($-CH_2CH_2-$) or a <u>cis</u>-vinylene group (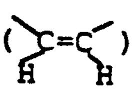), $C_{13}-C_{14}-C_{15}$ represents

(i) a group of the formula:

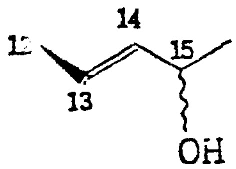

(V)

when [A] represents a group of the formula (II) or (III), or (ii) a group of the formula:

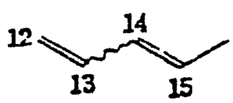

(VI)

when [A] represents a group of the formula (IV),

R represents (i) a group of the formula:

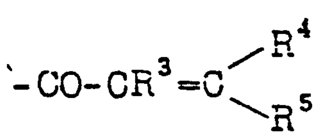

or

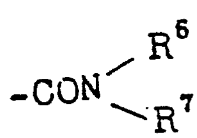

(wherein $R^3$, $R^4$, $R^5$, $R^6$ and $R^7$, which may be the same or different, each represents a hydrogen atom or a straight-chain or branched-chain alkyl group of 1-4 carbon atoms) when [A] represents a group of the formula (II) or (III), or

(ii) a hydrogen atom or a group of the formula:

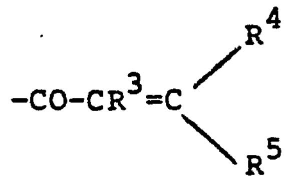 , 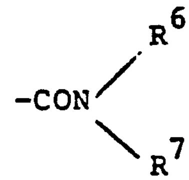 or $-COR^8$

(wherein $R^8$ represents a straight-chain or branched-chain alkyl group of 1-4 carbon atoms and $R^3$, $R^4$, $R^5$, $R^6$ and $R^7$ are as hereinbefore defined) when [A] represents a group of the formula (IV), $R^1$ represents a single bond or a straight-chain or branched-chain alkylene group of 1-5 carbon atoms, $R^2$ represents a straight-chain or branched-chain alkyl group of 1-8 carbon atoms, a cycloalkyl group of 4-7 carbon atoms either unsubstituted or substituted

by at least one straight-chain or branched-chain alkyl group of 1-8 carbon atoms, or a phenyl or phenoxy group either unsubstituted or substituted by at least one halogen atom, trifluoromethyl group or straight-chain or branched-chain alkyl group of 1-4 carbon atoms, the double bond between $C_9$-$C_{10}$ in the formulae (III) and (IV) is Z, the double bond between $C_{13}$-$C_{14}$ in the formula (V) is E, the double bonds between $C_{12}$-$C_{13}$ and between $C_{14}$-$C_{15}$ in the formula (VI), which may be in the same or different configurations are E, Z or a mixture thereof (i.e., EZ), with the proviso that when $R^1$ represents a single bond, $R^2$ does not represent a substituted or unsubstituted phenoxy group], and cyclodextrin clathrates thereof.

In the compounds of the general formula (I), there are several asymmetric carbon atoms. For example, in the general formula (I), when [A] is a group of the formula (II) and $C_{13}$-$C_{14}$-$C_{15}$ is a group of the formula (V), there are four asymmetric carbon atoms (at the 8-, 9-, 12- and 15-positions); when [A] is a group of the formula (III) and $C_{13}$-$C_{14}$-$C_{15}$ is a group of formula (V), the carbon atoms at the 8-, 12- and 15-positions are asymmetric; and when [A] is a group of formula (IV) and $C_{13}$-$C_{14}$-$C_{15}$ is a group of formula (VI), the carbon atoms at the 8-position is asymmetric. Further, asymmetric carbon atoms may occur in groups represented by $R^1$, $R^2$, $R^3$, $R^4$, $R^5$, $R^6$, $R^7$ and $R^8$. It is to be understood that all isomers and mixture thereof arising

from the presence of asymmetric carbon atoms are to be considered within the scope of formula I.

In the general formula (I), the alkylene groups of 1-5 carbon atoms represented by $R^1$ include methylene, ethylene, trimethylene, tetramethylene and pentamethylene groups and isomers thereof; $R^1$ preferably represents a single bond or a methylene or ethylene group.

In the general formula (I), the alkyl groups of 1-8 carbon atoms represented by $R^2$ include methyl, ethyl, propyl, butyl, pentyl, hexyl, heptyl and octyl groups and isomers thereof; butyl, pentyl or hexyl groups either unsubstituted or substituted by one or two methyl or ethyl groups are preferred; pentyl and 2-methylhexyl are especially preferred.

In the general formula (I), the substituted or unsubstituted cycloalkyl groups represented by $R^2$ include cyclobutyl, cyclopentyl, cyclohexyl, and cycloheptyl groups, and cyclobutyl, cyclopentyl, cyclohexyl and cycloheptyl groups substituted by one or more methyl, ethyl, propyl, butyl, pentyl, hexyl, heptyl and octyl groups and isomers thereof; cyclobutyl, cyclopentyl or cyclohexyl groups either

unsubstituted or substituted by one methyl, ethyl, propyl or butyl group are preferred; 3-propylcyclopentyl is especially preferred.

In the general formula (I), the substituted or unsubstituted phenyl or phenoxy groups represented by $R^2$ include phenyl and phenoxy groups, and phenyl and phenoxy groups substituted by one or more atoms or groups selected from the fluorine and chlorine atoms and trifluoromethyl, methyl, ethyl, propyl or butyl groups; phenyl or phenoxy groups either unsubstituted or substituted by one chlorine atom, trifluoromethyl group, methyl or ethyl group are preferred; chlorine is a preferred substituent.

In the general formula (I), preferred groupings $R^1$-$R^2$ are butyl, pentyl, 1-methylpentyl, 2-methylpentyl, 3-methylpentyl, 1,1-dimethylpentyl, 1-ethylpentyl, 2-ethylpentyl, hexyl, 1-methylhexyl, 2-methylhexyl, 1-ethylhexyl, 2-ethylhexyl, heptyl, 2-methylheptyl, 2-ethylheptyl, cyclobutyl, 1-propylcyclobutyl, 1-butylcyclobutyl, 3-ethylcyclobutyl, 3-propylcyclobutyl, cyclopentyl, cyclopentylmethyl, 2-cyclopentylethyl, 3-ethylcyclopentyl, 3-propylcyclopentyl, 3-butylcyclopentyl, cyclohexyl, cyclohexylmethyl, 2-cyclohexylethyl, 4-methylcyclohexyl, 4-ethylcyclohexyl, 4-propylcyclohexyl, 4-butylcyclohexyl, benzyl, 2-phenylethyl, 4-methylbenzyl, 4-ethylbenzyl, phenoxymethyl, 2-phenoxyethyl, 3-chlorophenoxymethyl, 4-chlorophenoxymethyl, 3-trifluoromethylphenoxymethyl, 4-trifluoromethylphenoxymethyl, 4-methylphenoxymethyl and 4-ethylphenoxymethyl; pentyl, 2-methylhexyl, 3-propylcyclopentyl and 3-chlorophenoxymethyl are especially preferred.

In the general formula (I), the alkyl groups of 1-4 carbon atoms represented by $R^3$, $R^4$, $R^5$, $R^6$, $R^7$ and $R^8$ include methyl, ethyl, propyl and butyl groups, and isomers thereof; $R^3$, $R^4$ and $R^5$ preferably represent a hydrogen atom or a methyl or ethyl group; both $R^6$ and $R^7$ preferably represent a hydrogen atom or a methyl or ethyl group; and $R^8$ preferably represents a methyl or ethyl group.

In the general formula (I), R preferably represents a hydrogen, acryloyl, 2-methylacryloyl, 2-ethylacryloyl, 3-methylacryloyl, 3-ethylacryloyl, 3,3-dimethylacryloyl, 3,3-diethylacryloyl, 2,3-dimethylacryloyl, 2,3-diethylacryloyl, 2,3,3-trimethylacryloyl, 2,3,3-triethylacryloyl, carbamoyl, dimethylcarbamoyl, diethylcarbamoyl, acetyl or propionyl; the hydrogen atom, 3,3-dimethylacryloyl, carbamoyl, dimethylcarbamoyl and acetyl are especially preferred.

Further, in the general formula (I), X is preferably a cis-vinylene group, and in the general formula (I), where $C_{13}-C_{14}-C_{15}$ represents a group of the formula (V), the preferred configuration of the hydroxyl group attached to the carbon atom at the 15-position is the α-configuration.

As mentioned hereinbefore, the compounds of the present invention may be designated as PGD derivatives. For example, typical examples of the compounds of the present invention represented by the formulae:

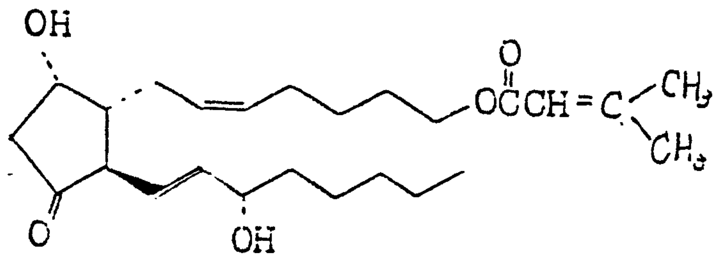

a

and

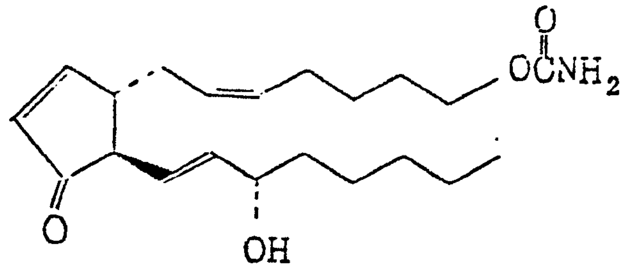

b

may be designated, respectively, as 1-O-(3,3-dimethylacryloyl)-$PGD_2$ alcohol and 1-O-carbamoyl-9-deoxy-$\Delta^9$-$PGD_2$ alcohol.

Further, all the compounds of the present invention may be designated systematically as derivatives of a prostane represented by the formula:

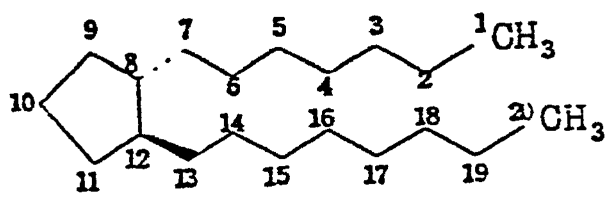

In this case, the compounds represented by formulae a and b, and the compound represented by the formula:

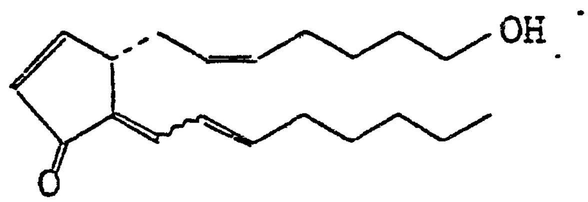

c

may be designated, respectively, as (5Z,13E)-(9S,15S)-1-O-(3,3-dimethylacryloyl)-11-oxoprosta-5,13-diene-1,9,15-triol, (5Z, 13E)-(15S)-1-O-carbamoyl-11-oxoprosta-5,9,13-triene-1,15-diol and (5Z,14EZ)-11-oxoprosta-5,9,12,14-tetraen-1-ol.

The compounds of the present invention represented by the general formula (I) comprise the 1-O-acryloyl-PGD alcohol and 1-O-carbamoyl-PGD alcohol derivatives represented by the general formula:

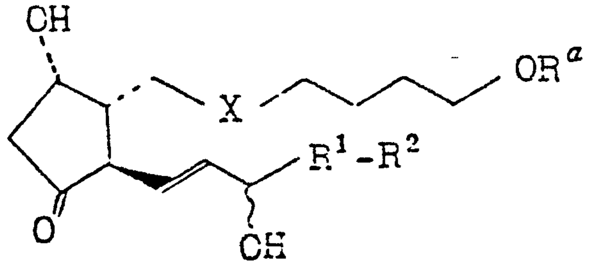

(I*a*)

wherein $R^a$ represents a group $-CO-CR^3=C\begin{smallmatrix}R^4\\R^5\end{smallmatrix}$ or $-CON\begin{smallmatrix}R^6\\R^7\end{smallmatrix}$ (wherein all of the symbols are as hereinbefore defined) and the other symbols are as hereinbefore defined,

and the 1-O-acryloyl-9-deoxy-$\Delta^9$-PGD alcohol and 1-O-carbamoyl-9-deoxy-$\Delta^9$-PGD alcohol derivatives represented by the general formula:

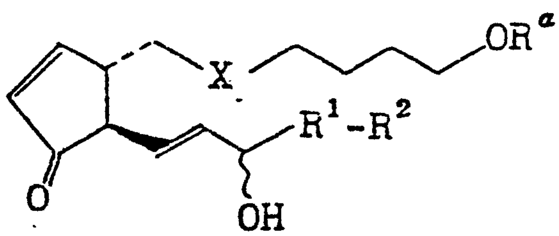

(I*b*)

(wherein all of the symbols are as hereinbefore defined)

and the compounds represented by the general formula:

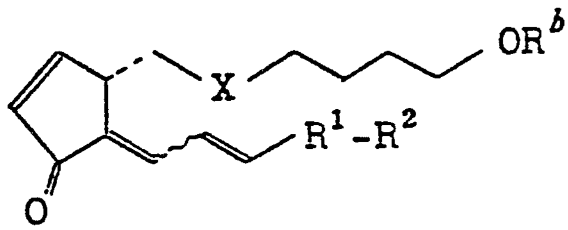

(Ic)

wherein $R^b$ represents a hydrogen atom or a group $-CO-CR^3=C\genfrac{}{}{0pt}{}{R^4}{R^5}$, $-CON\genfrac{}{}{0pt}{}{R^6}{R^7}$ or $-COR^8$ (wherein all of the symbols are as hereinbefore defined) and the other symbols are as hereinbefore defined.

According to a feature of the present invention the PGD derivatives of the general formula (Ic) may be prepared by reacting a compound of the general formula:

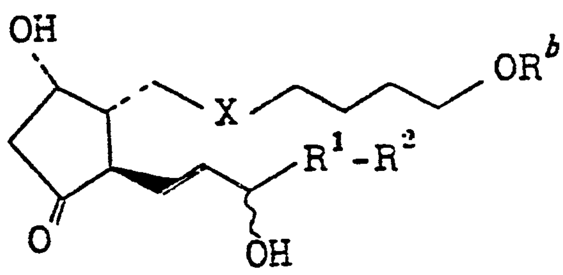

(VII)

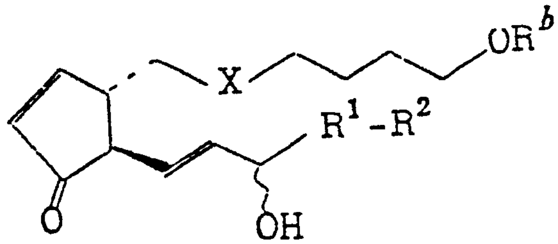

(VIII)

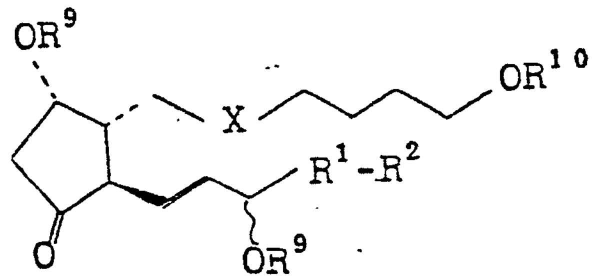

(IX)

or

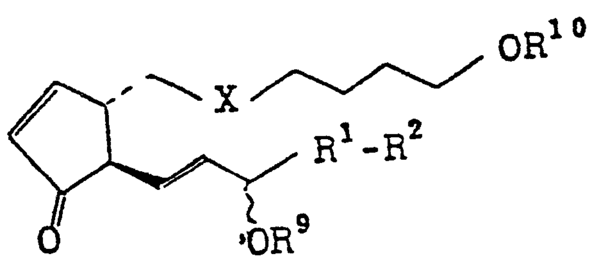

(X)

(wherein $R^9$ represents a tetrahydropyran-2-yl group or tetrahydrofuran-2-yl group either unsubstituted or substituted by at least one alkyl group, or a 1-ethoxyethyl group, preferably a tetrahydropyran-2-yl group; $R^{10}$ represents a group $R^9$ or a group of the formula:

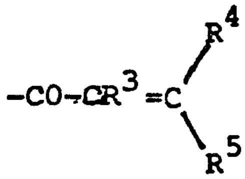 , 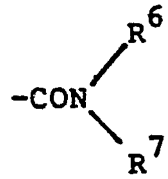 or $-COR^8$ (wherein all of the symbols are as hereinbefore defined) and the other symbols are as hereinbefore defined), with an aqueous acid, preferably in an inert organic solvent, for example, methylene chloride, chloroform, carbon tetrachloride, diethyl ether, N,N-dimethylformamide, tetrahydrofuran or a mixture of two or more such solvents, using as the aqueous acid, for example, an aqueous solution of an inorganic acid, e.g. hydrochloric acid, hydrobromic acid, sulfuric acid or phosphoric acid, or an organic acid, e.g. acetic acid, propionic acid or oxalic acid, at the reflux temperature of the solvent. The reaction is preferably conducted in tetrahydrofuran using 1N hydrochloric acid at the reflux temperature of the reaction mixture.

The compounds of the general formula (VII) include the PGD analogues represented by the general formula:

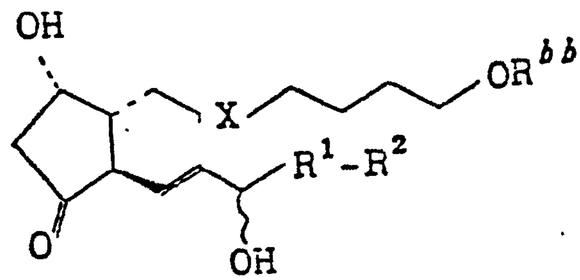

(VIIa)

(wherein $R^{bb}$ represents a hydrogen atom or a group $COR^8$ as hereinbefore defined and the other symbols are as hereinbefore defined); the compounds of the general formulae (Ia) and (VIIa) may be prepared by the hydrolysis, under acidic conditions, of a compound represented by the general formula:

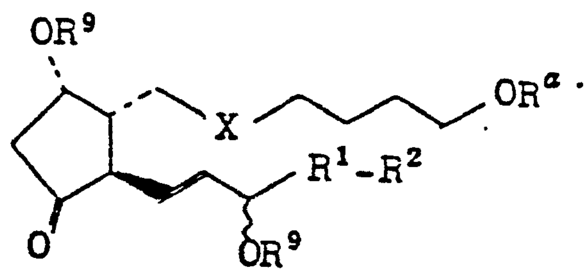

(IXa)

or

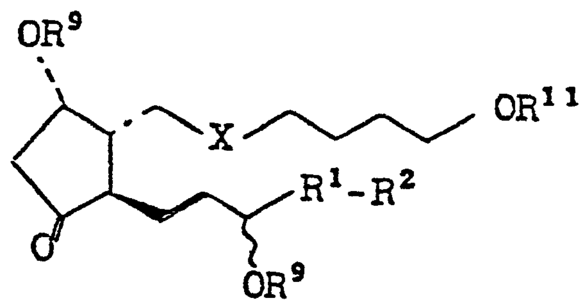

(IXb)

(wherein $R^{11}$ represents a group $R^9$ or a group of the formula $-COR^8$ as hereinbefore defined and the other symbols are as hereinbefore defined), to convert to hydroxy groups the groups $OR^9$ and, when $OR^{11}$ represents a group $OR^9$, the group $OR^{11}$. This hydrolysis may be conducted for example:

(1) In an aqueous solution of an organic acid such as acetic acid, propionic acid, oxalic acid or p-toluenesulfonic acid or an aqueous solution of an inorganic acid such as hydrochloric acid, sulfuric acid or phosphoric acid, suitably in the presence of a water-miscible organic solvent, for example, a lower alkanol (preferably methanol) or an ether such as 1,2-dimethoxyethane or tetrahydrofuran (preferably tetrahydrofuran) at a temperature of from room temperature to 75°C (preferably 45°C or lower); or,

(2) In an anhydrous lower alkanol such as methanol or ethanol, in the presence of an organic acid such as p-toluenesulfonic acid or trifluoroacetic acid at a temperature of 10-45°C.

The hydrolysis is preferably conducted using a mixture of dilute hydrochloric acid and tetrahydrofuran, a mixture of dilute hydrochloric acid and methanol, a mixture of acetic acid, water and tetrahydrofur-

an, a mixture of phosphoric acid, water and tetrahydrofuran, or a mixture of p-toluenesulfonic acid and absolute methanol.

The compounds of the general formula (VIII) include the PGD analogues represented by the general formula (Ib) and the PGD analogues represented by the general formula:

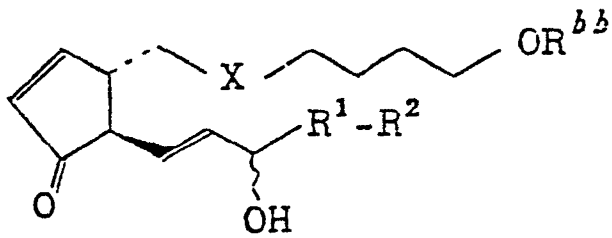

(VIIIa)

(wherein all of the symbols are as hereinbefore defined). The PGD compounds of the general formulae (Ib) and (VIIIa) may be prepared by the hydrolysis, under acidic conditions, of the compounds of the general formulae:

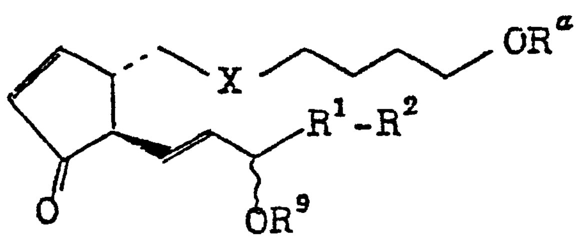

(Xa)

and

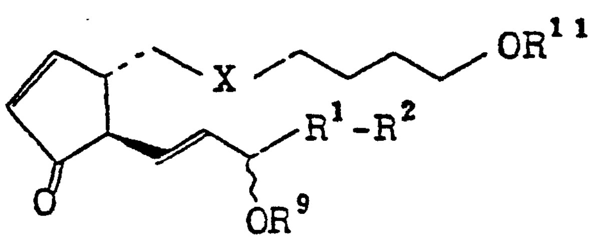

(Xb)

(wherein all of the symbols are as hereinbefore defined) to convert to hydroxy groups the group $OR^9$ and, when $OR^{11}$ represents a group $OR^9$, the group $OR^{11}$. This hydrolysis is preferably carried out as hereinbefore described for the preparation of the compounds represented by the general formulae (Ia) and (VIIa).

Scheme A shows a series of the above-mentioned reactions to produce the PGD analogues of the general formulae (Ia), (Ib) and (Ic), wherein all of the symbols are as hereinbefore defined.

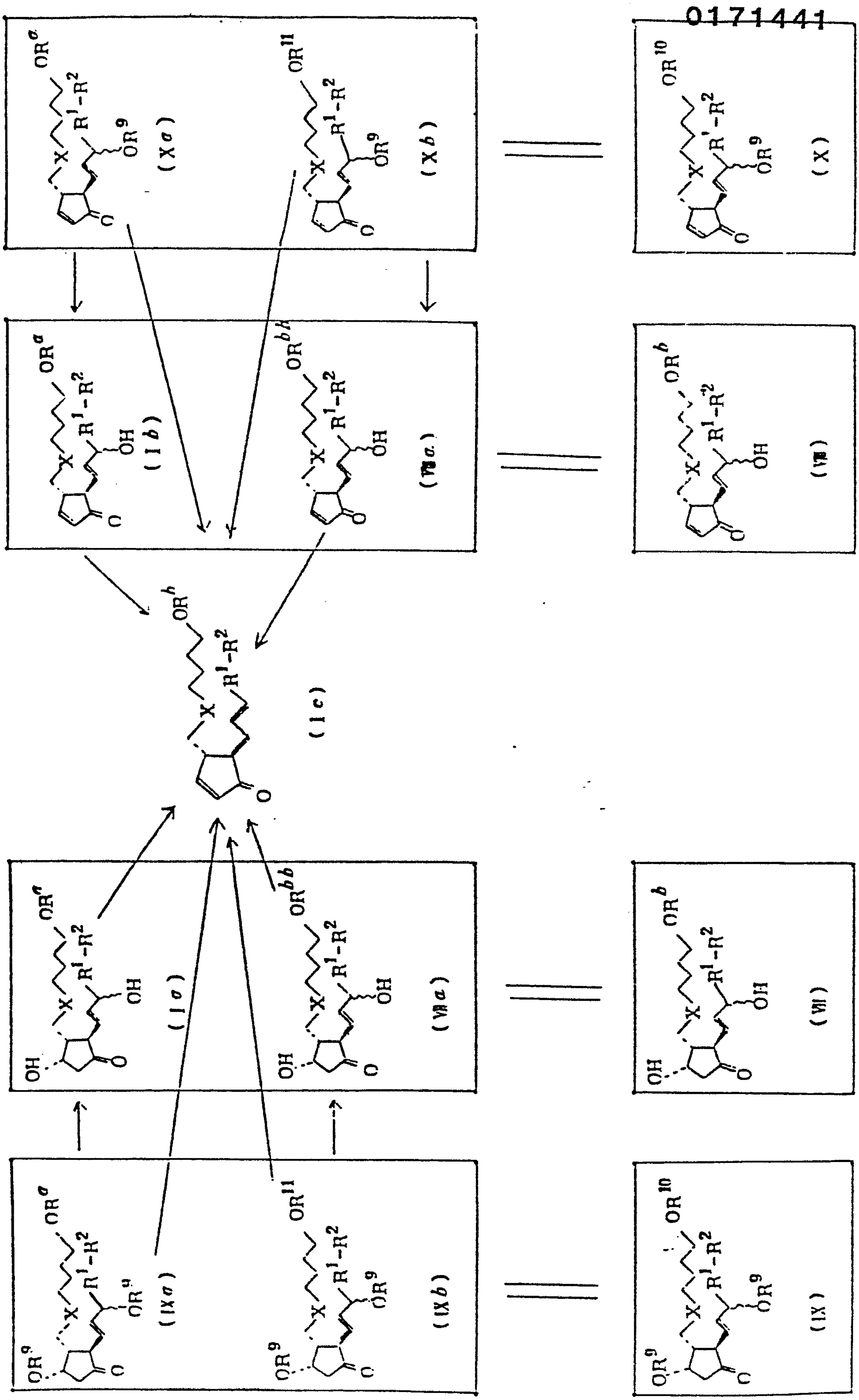

(Xa) (Xb) (X)

(Ib) (VIIIa) (VIII)

(Ic)

(Ia) (VIIa) (VII)

(IXa) (IXb) (IX)

According to a further feature of the present invention, the compounds of the general formula (I), wherein R represents the group represented by the formula $-CO-CR^3=C{<}^{R^4}_{R^5}$ (wherein all of the symbols are as hereinbefore defined), that is, the compounds represented by the general formula:

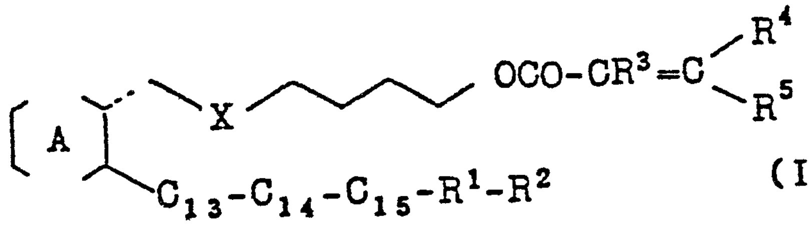

(I*d*)

(wherein all of the symbols are as hereinbefore defined) may be prepared by reacting a compound of the general formula:

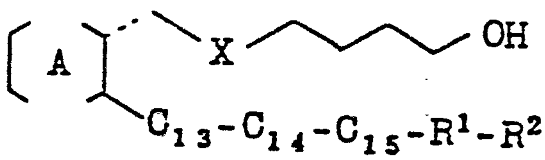

(XI)

(wherein all of the symbols are as hereinbefore defined) with an acid halide of the general formula:

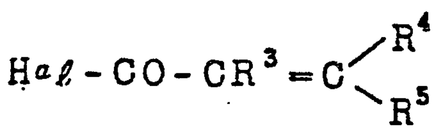

(XII)

(wherein Hal represents a chlorine atom, a bromine atom or an

iodine atom, and the other symbols are as hereinbefore defined) to convert the hydroxy group attached to the 1-position to a group $OCO-CH^3=C\langle^{R4}_{R5}$. The selective acylation is preferably conducted at a temperature not higher than room temperature, most preferably at 0°C to -20°C, in an inert organic solvent, in the presence of a tertiary amine such as pyridine or triethylamine.

In a similar manner, the compound of the general formula (I) wherein R represents the formula $-COR^8$ (wherein $R^8$ is hereinbefore defined), that is, the compound represented by the general formula:

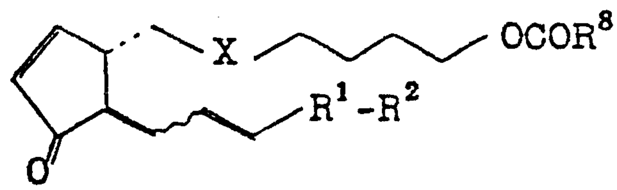

(I*e*)

(wherein all of the symbols are as hereinbefore defined), and the compounds represented by the general formulae:

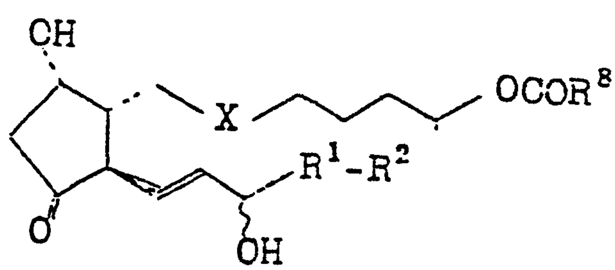

(VIIb)

and

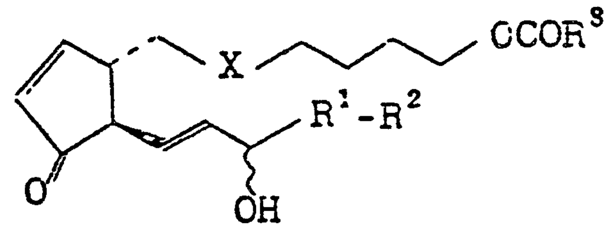

(VIII*b*)

(wherein all of the symbols are as hereinbefore defined) among the compounds of the general formulae (VII) and (VIII) may also be prepared by reacting the compounds represented, respectively, by the formulae:

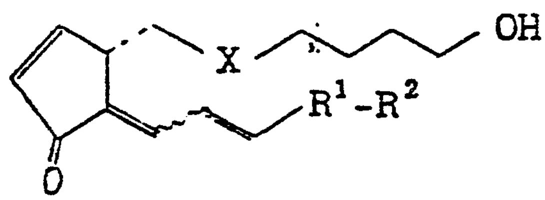

(XI*a*)

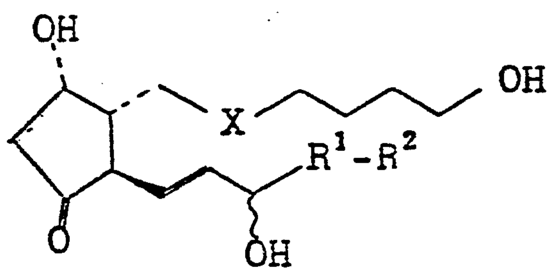

(XI*b*)

and

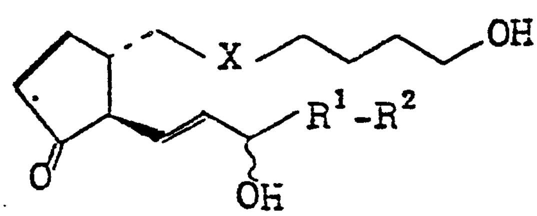

(XI*c*)

(wherein all of the symbols are as hereinbefore defined) with an acid halide represented by the general formula:

$$Hal - CO - R^8 \quad (XIII)$$

(wherein all of the symbols are as hereinbefore defined) to convert the hydroxy group attached to the 1-position to a group $-OCOR^8$.

The compounds represented by the general formulae (XIa), (XIb) and (XIc), namely (XI), which are employed as starting materials in the above-mentioned acylation are compounds covered by the the general formulae (Ic), (VIIa) or (VIIIa).

The compounds represented by the general formulae (IX) and (X) may be prepared, respectively, according to the series of reaction processes illustrated in Schemes B and C. In these schemes, $R^{12}$ represents a trimethylsilyl group, triethylsilyl group, tributylsilyl group, tert-butyl dimethylsilyl group, or tribenzylsilyl group, or other tri-substituted silyl group such as a triphenylsilyl group, preferably the tert-butyldimethylsilyl group, and T represents an alkylsulfonyl group or a substituted or unsubstituted arylsulfonyl group, preferably a mesyl group or a tosyl group and the other symbols are as hereinbefore defined.

Scheme B

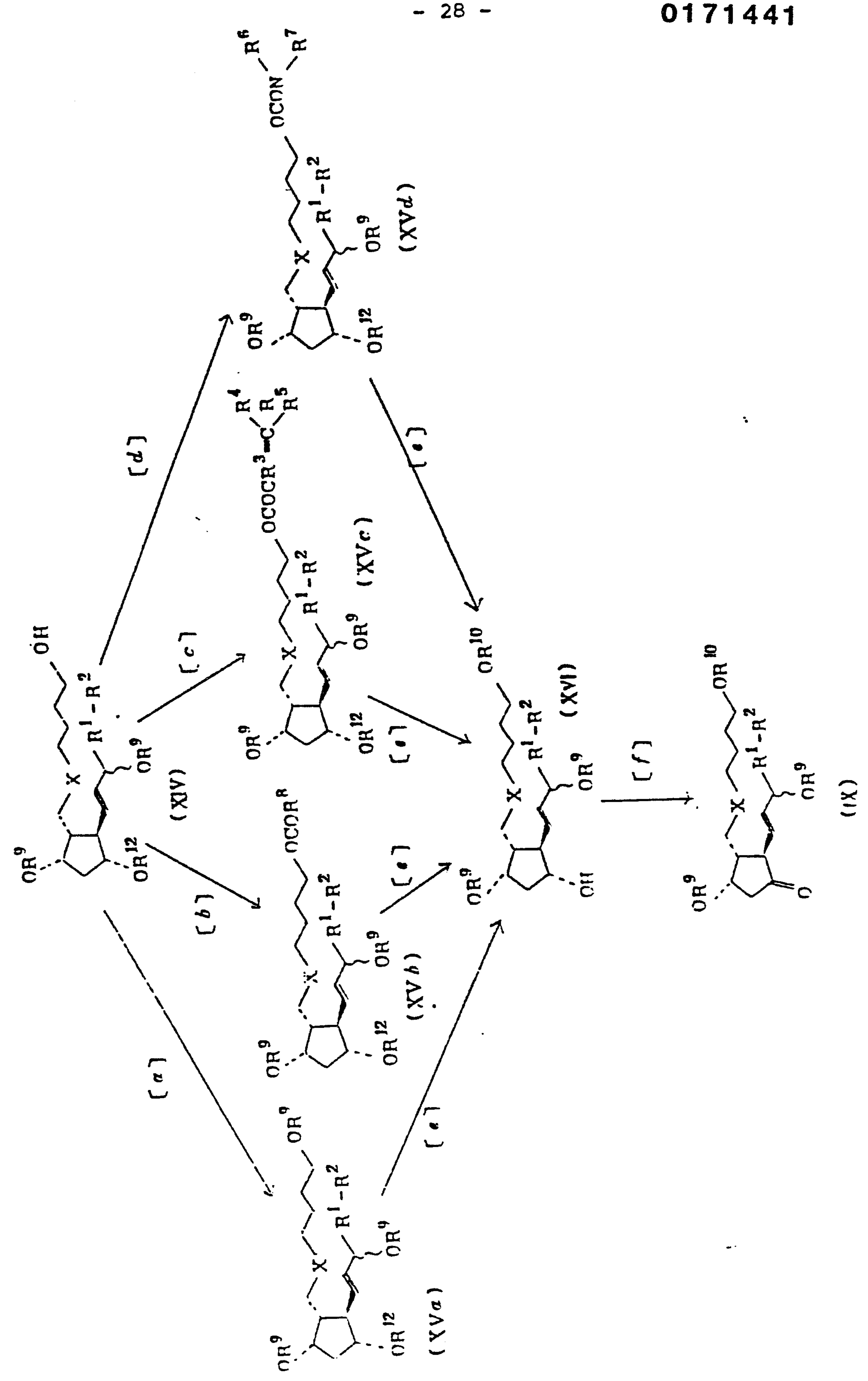

0171441

Scheme C

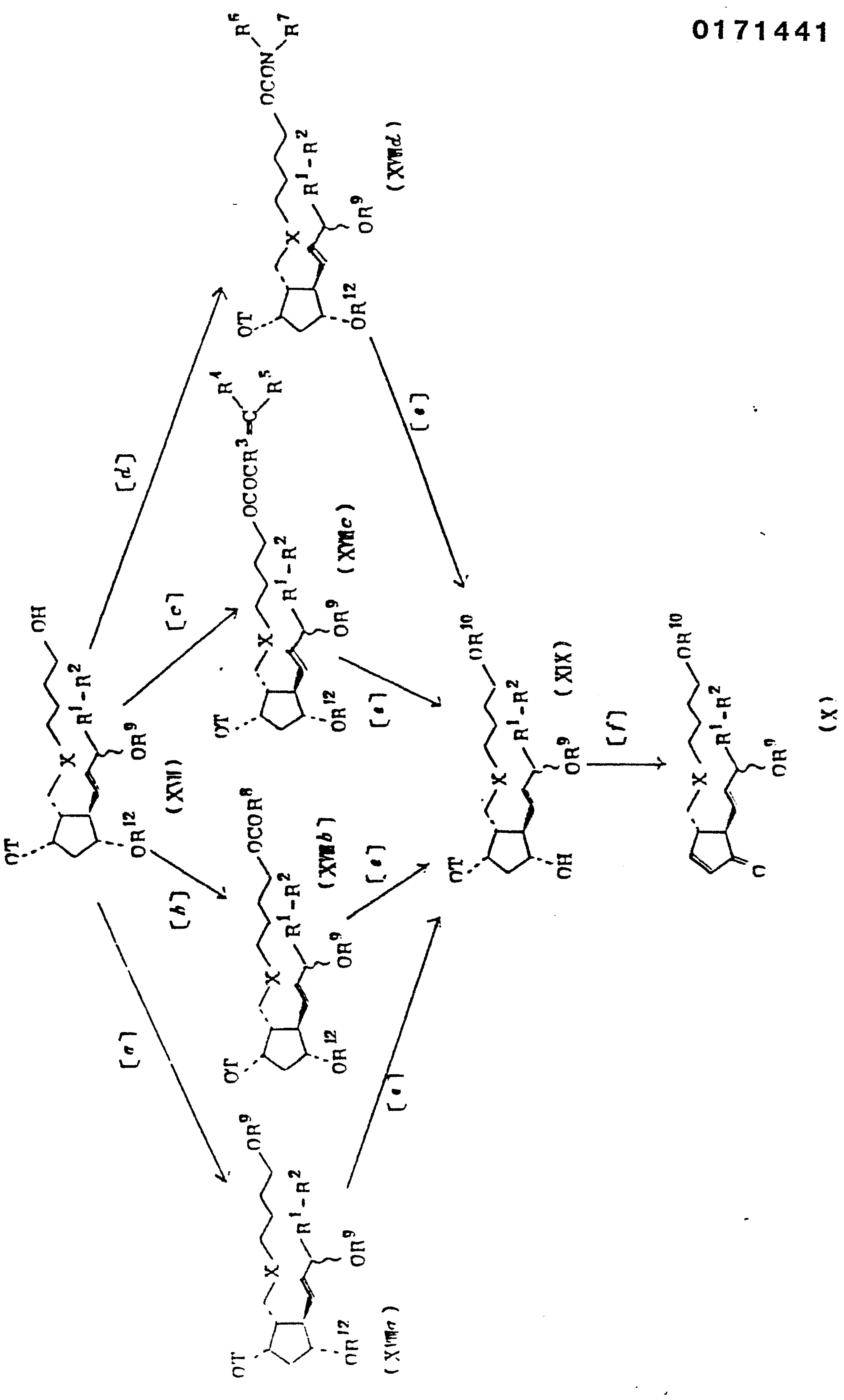


(XVII) (XVIIIa) (XVIIIb) (XVIIIc) (XVIIId) (XIX) (X)

All of the steps in Schemes B and C may be carried out by known methods. Step (a), to protect a hydroxyl group, may be conducted using e.g. 2,3-dihydropyran, 2,3-dihydrofuran, or ethyl vinyl ether, in an inert organic solvent, for example, methylene chloride, chloroform or diethyl ether, in the presence of a condensing agent, for example, p-toluenesulfonic acid, sulfuric acid or trifluoroacetic acid at a temperature of from room temperature to -30°C. Preferably, it is conducted using 2,3-dihydropyran in methylene chloride in the presence of pyridine p-toluenesulfonate or p-toluenesulfonic acid at room temperature.

The steps (b) and (c), are acylations which may be carried out as hereinbefore described for the preparation of the compounds represented, respectively, by the general formulae (Ie) and (Id).

Step (d) is a carbamoylation reaction, which may be conducted by reacting the compounds of the general formula (XIV) or (XVII) with phenyl chloroformate at a temperature of 0°C, in an inert organic solvent such as methylene chloride, tetrahydrofuran, dimethyl ether or N,N-dimethylformamide (preferably dimethyl ether), in the presence of a base such as triethylamine or pyridine, and then with liquid ammonia or a compound represented by the formula $HN\langle^{R^6}_{R^7}$, wherein $R^6$ and $R^7$ are as hereinbefore described, at a temperature of -78°C.

The step (e) is a reaction in which the $OR^{12}$ group is converted into a hydroxyl group. This reaction may be carried out as described in "Protective Groups in Organic Synthesis" published by John Wiley & Sons, Inc.(USA), page 39-50 (1981) (hereinafter referred to as "Reference A"). This reaction is preferably conducted by using tetrabutylammonium fluoride ($n\text{-}Bu_4N^+F^-$) in tetrahydrofuran at room temperature.

The step (f) is a reaction to convert the hydroxy group into an oxo group and, in addition to eliminate the OT group to form a double bond between $C_9$-$C_{10}$. Such an oxidation reaction is well known, and is described in detail in, for example,

(a) "Synthetic Organic Chemistry III, Organic Synthesis 1", pp. 176-206 (compiled by Tetsuji Kameya and published by Nankodo (Japan) on Aug. 1, 1976), or,

(b) "Compendium of Organic Synthetic Methods", vol. 1, vol. 2, and vol.3, section 48 or 168 (published by John Wiley & Sons, Inc. (USA) in 1971, 1974, and 1977, respectively).

The oxidation is preferably carried out under mild neutral conditions using, for example, dimethyl-sulphide-N-chlorosuccinimide complex, thioanisole-N-chlorosuccinimide complex dimethylsulphide-chlorine complex, thioanisole-chlorine complex (see J. Amer. Chem. Soc., 94, 7586 (1972) with

respect to these complexes), dicyclohexylcarbodiimide-dimethylsulphoxide complex (see J. Amer. Chem. Soc., 87, 5661 (1965), pyridinium chlorochromate ($C_5H_5NHCrO_3Cl$) (see Tetrahedron Letters, 2647 (1975)), sulphuric anhydride-pyridine complex (see J. Amer. Chem. Soc., 89, 5505 (1967)), chromyl chloride (see. J. Amer. Chem. Soc., 97, 5929 (1975)), chromium trioxide-pyridine complex (for example, Collins' reagent), Jones' reagent (chromic acid solution, prepared from chromium trioxide, manganese sulfate, sulfuric acid and water), or oxalyl chloride and dimethylsulfoxide (i.e. Swern oxidation); suitably the Collins oxidation, Jones oxidation or Swern oxidation may be employed. The Collins' oxidation may be conducted in a halogenated hydrocarbon such as chloroform, methylene chloride or carbon tetrachloride at a temperature of from room temperature to 0°C. The Jones oxidation is generally conducted at a temperature of not higher than room temperature. The Swern oxidation may be conducted by reaction in a halogenated hydrocarbon such as chloroform or methylene chloride at a temperature of from -50°C to -60°C, and then treatment with triethylamine.

The compounds of the general formulae (XIV) and (XVII) in Schemes B and C, which are employed as starting materials, may be prepared according to the sequence of reaction steps illustrated in Scheme D, wherein all of the symbols are as hereinbefore defined.

Scheme D

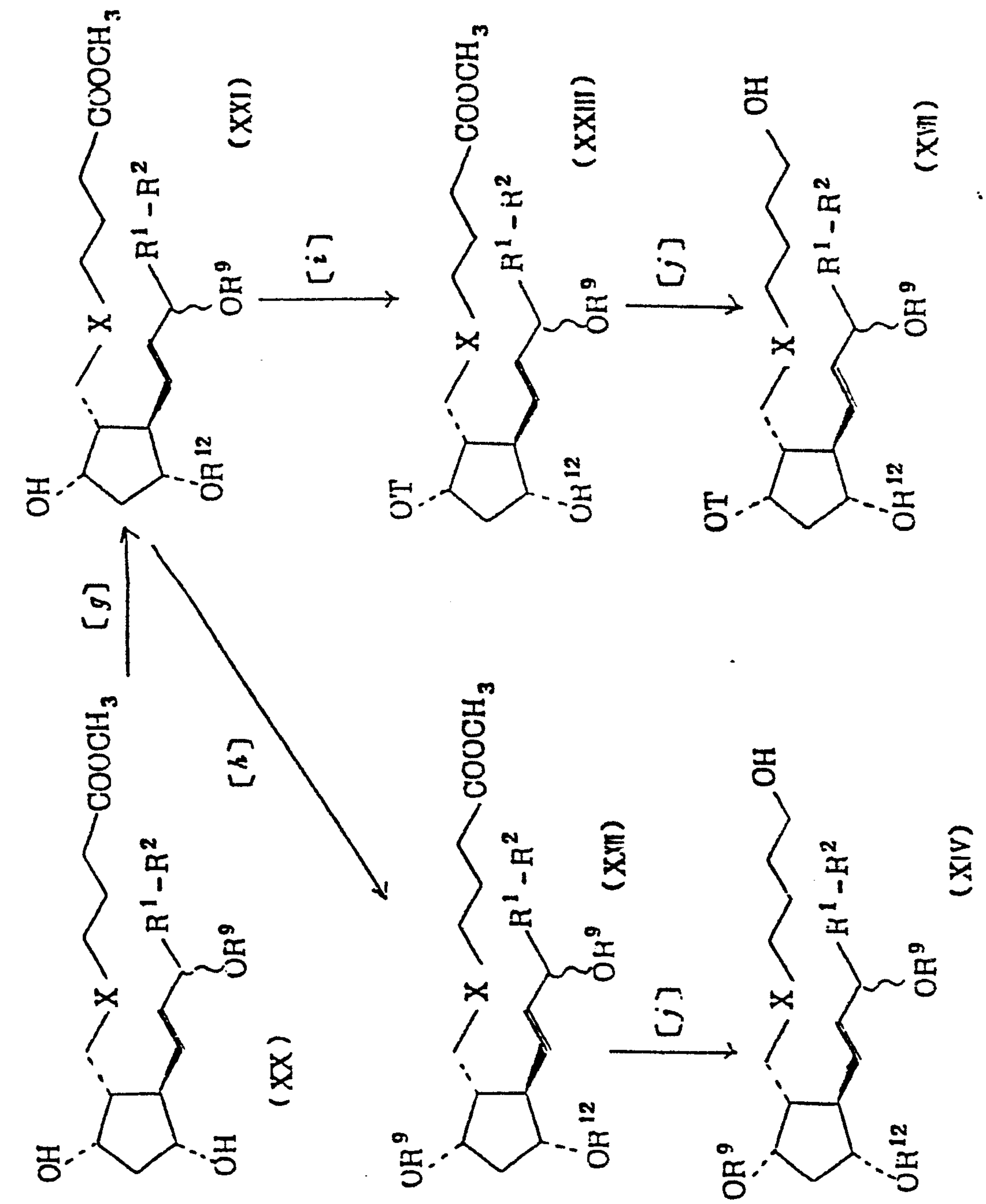

All the steps in Scheme D are conducted by known methods. Step (g), for example, is a reaction in which the hydroxyl group at the 11-position is selectively converted into $OR^{12}$. Such reactions are described in detail in Reference A. Preferably, it is conducted by reaction with a chlorosilane compound represented by the formula $Cl-R^{12}$ (wherein $R^{12}$ is as hereinbefore defined), in dimethylaminopyridine or dimethylformamide, in the presence of a base such as pyridine, imidazole or triethylamine, at a temperature of from room temperature to 50°C. The reaction must be carried out carefully to avoid conversion of the hydroxyl group attached to the 9-position into a group $OR^{12}$.

The step (h) is a reaction in which the hydroxyl group attached to the 9-position is converted into a group $OR^{9}$. This step may be conducted as in the step (a).

In step (i) the hydroxyl group attached to the 9-position is converted into a group OT. This reaction may be conducted by reaction with an alkylsulfonyl chloride such as mesyl chloride or arylsulfonyl chloride such as tosyl chloride (i) in an inert organic solvent such as methylene chloride, in the presence of a tertiary amine such as pyridine or triethylamine or (ii) in pyridine,

at a temperature of from -30°C to 50°C.

The step (j) is a reducing reaction in which the $-COOCH_3$ group is converted into the $-CH_2OH$ group. This reaction may be conducted by any suitable reducing agent, preferably diisobutylaluminium hydride (DIBAL) in an inert organic solvent such as hexane, tetrahydrofuran or toluene, at a temperature of from 0°C to -30°C.

The compounds of the general formula (XX) are known compounds and disclosed as chemical products represented by the general formula (XXIII) in the specification of European Patent Publication No. 0098141.

Cyclodextrin clathrates of the prostaglandin D analogues of general formula (I) can be prepared using α- β- or γ-cyclodextrins or a mixture thereof, using the procedure described in Japanese Patent No. 790979 and Japanese Patent Application OPI No. 39057/72. Conversion into the cyclodextrin clathrate serves to increase the stability of the prostaglandin D analogues of the general formula (I).

The prostaglandin D analogues of the general formula (I) and their cyclodextrin clathrates have a specific strong anti-tumor effect, and moreover their toxicity is extremely low. They may therefore be employed as very effective anti-tumor agents in the prevention or therapy of leukemia and solid cancer, and in the treatment to produce remission thereof.

Furthermore, prostaglandin D analogues of the general formula (I) have the effect of increasing the number of white blood cells. The compounds of the present invention may therefore be used as a preventive and therapeutic agent against infection by such microorganisms as bacteria and viruses. Compounds of the present invention are also effective on leukocyto-penia induced by administration of common anti-tumor agents, because anti-tumor agents generally cause a decrease in the number of white blood cells as a side effect.

In an *in vitro* test on the inhibition of proliferation of cells originated from human mouth tumor (KB-cells) the compounds of the present invention showed an excellent anti-tumor effect. The experimental methods and the results are described below.

Test on Inhibition of Proliferation Using Cells Originated from Human Mouth Tumor (KB-cells)

(Experimental Method)

The cells originated from human mouth tumor (KB-cells) were added to Eagle's MEM culture solution containing 10% bovine fetal serum, the number of cells in the culture solution was adjusted to 1 x $10^5$ cells/ml, a solution in ethanol of the compound of the present invention was added to give a concentration of 5μg/ml or less (3 compounds) and the mixture subjected to stationary

culture at 37°C for 4 days. As a control, a culture solution containing 0.1% of ethanol was similarly cultured. After the culture, the cells were stained by the Trypan Blue staining method, and the surviving cell number was measured to determine the $IC_{50}$ value from the degree of inhibition relative to the control. The results are given below. Compounds are identified by the Example (described hereinafter) in which they are prepared.

Inhibition of Proliferation Using Cells Originated from Human Mouth Tumor (KB-Cells)

| Test Compound | Inhibition of Proliferation ($IC_{50}$, μg/ml) |
|---|---|
| Compound 1 | 2.1 |
| Compound 1(c) | 1.1 |
| Compound 2 | < 1 |
| Compound 3 | 1.6 |

In an *in vivo* test on increase of white blood cells using blood collected from male mice, the compounds of the present invention showed an excellent effect. The experimental method and results are described below.

Test on increase of white blood cells

(Experimental Method)

A solution of a compound of the present invention in ethanol was diluted with 1% Tween 80 (registered Trade

Mark) and the solution was intraperitoneally administered to ICR male mice of 6-7 weeks old once a day for 14 days. On the day following the final administration, the blood was collected by cardiac puncture with Anglot ® (containing heparin and EDTA) as an anticoagulant. The blood was diluted with Seruento (registered Trade Mark), and the numbers of white blood cells and red blood cells were counted by Micro cell counter (model CC-120).

The results are given below.

Effect on hematological test

| Dose (mg/kg: ip) | WBC ($x10^2/mm^3$) | RBC ($x10^4/mm^3$) |
|---|---|---|
| control | 66 ± 4.6 | 872 ± 15.8 |
| Example 2,2(a) | | |
| 20 | 299 ± 78.5 | 571 ± 64.5 |
| | ($p < 0.01$ | ($p < 0.001$) |

WBC : white blood cell

RBC : red blood cell

As shown in the table, although red blood cells decrease about 0.65 times, white blood cells increase about 4.5 times at the dose of 20 mg/kg as compared with control. As demonstrated by the results in the table, compounds of the present invention have the effect of increasing the number of white blood cells *in vivo*.

On the other hand, it was confirmed that the acute toxicity of the compounds of the present invention was more than 75 mg/kg by intraperitoneal administration. Therefore, PGD derivatives of the present invention may be considered to be sufficiently safe and suitable for medical use.

Preferred compounds of the general formula (I) of the present invention are, for example, as follows:

1-O-(3,3-dimethylacryloyl)-$PGD_2$ alcohol,

1-O-(3,3-dimethylacryloyl)-16-methyl-$PGD_2$ alcohol,

1-O-(3,3-dimethylacryloyl)-16,16-dimethyl-$PGD_2$ alcohol,

1-O-(3,3-dimethylacryloyl)-17,20-dimethyl-$PGD_2$ alcohol,

1-O-(3,3-dimethylacryloyl)-15-(1-butylcyclobutyl)-16,17,18,19,20-pentanor-$PGD_2$ alcohol,

1-O-(3,3-dimethylacryloyl)-15-cyclopentyl-16,17,18,19,20-pentanor-$PGD_2$ alcohol,

1-O-(3,3-dimethylacryloyl)-15-(3-propylcyclopentyl)-16,17,18,19,20-pentanor-$PGD_2$ alcohol,

1-O-(3,3-dimethylacryloyl)-15-(4-butylcyclohexyl)-16,17,18,19,20-pentanor-$PGD_2$ alcohol,

1-O-(3,3-dimethylacryloyl)-16-phenyl-17,18,19,20-tetranor-$PGD_2$ alcohol,

1-O-(3,3-dimethylacryloyl)-16-phenoxy-17,18,19,20-tetranor-$PGD_2$ alcohol,

1-O-(3,3-dimethylacryloyl)-16-(3-chlorophenoxy)-17,18,19,20-tetranor-$PGD_2$ alcohol,

1-O-(3,3-dimethylacryloyl)-16-(3-trifluoromethylphenoxy)-17,18,19,20-tetranor-$PGD_2$ alcohol,

1-O-carbamoyl-$PGD_2$ alcohol,

1-O-carbamoyl-16-methyl-$PGD_2$ alcohol,

1-O-carbamoyl-16,16-dimethyl-$PGD_2$ alcohol,

1-O-carbamoyl-17,20-dimethyl-$PGD_2$ alcohol,

1-O-carbamoyl-15-(1-butylcyclobutyl)-16,17,18,19,20-pentanor-$PGD_2$ alcohol,

1-O-carbamoyl-15-cyclopentyl-16,17,18,19,20-pentanor-$PGD_2$ alcohol,

1-O-carbamoyl-15-(3-propylcyclopentyl)-16,17,18,19,20-pentanor-$PGD_2$ alcohol,

1-O-carbamoyl-15-(4-butylcyclohexyl)-16,17,18,19,20-pentanor-$PGD_2$ alcohol,

1-O-carbamoyl-16-phenyl-17,18,19,20-tetranor-$PGD_2$ alcohol,

1-O-carbamoyl-16-phenoxy-17,18,19,20-tetranor-$PGD_2$ alcohol,

1-O-carbamoyl-16-(3-chlorophenoxy)-17,18,19,20-tetranor-$PGD_2$ alcohol,

1-O-carbamoyl-16-(3-trifluoromethylphenoxy)-17,18,19,20-tetranor-$PGD_2$ alcohol,

1-O-(dimethylcarbamoyl)-$PGD_2$ alcohol,

1-O-(dimethylcarbamoyl)-16-methyl-$PGD_2$ alcohol,

1-O-(dimethylcarbamoyl)-16,16-dimethyl-$PGD_2$ alcohol,

1-O-(dimethylcarbamoyl)-17,20-dimethyl-$PGD_2$ alcohol,

1-O-(dimethylcarbamoyl)-15-(1-butylcyclobutyl)-16,17,18,19,20-pentanor-$PGD_2$ alcohol,

1-O-(dimethylcarbamoyl)-15-cyclopentyl-16,17,18,19,20-pentanor-$PGD_2$ alcohol,

1-O-(dimethylcarbamoyl)-15-(3-propylcyclopentyl)-16,17,18,19,20-pentanor-$PGD_2$ alcohol,

1-O-(dimethylcarbamoyl)-15-(4-butylcyclohexyl)-16,17,18,19,20-pentanor-$PGD_2$ alcohol,

1-O-(dimethylcarbamoyl)-16-phenyl-17,18,19,20-tetranor-$PGD_2$ alcohol.

1-O-(dimethylcarbamoyl)-16-phenoxy-17,18,19,20-tetranor-$PGD_2$ alcohol,

1-O-(dimethylcarbamoyl)-16-(3-chlorophenoxy)-17,18,19,20-tetranor-$PGD_2$ alcohol,

1-O-(dimethylcarbamoyl)-16-(3-trifluoromethylphenoxy)-17,18,19,20-tetranor-$PGD_2$ alcohol,

1-O-(3,3-dimethylacryloyl)-9-deoxy-$\Delta^9$-$PGD_2$ alcohol,

1-O-(3,3-dimethylacryloyl)-16-methyl-9-deoxy-$\Delta^9$-$PGD_2$ alcohol,

1-O-(3,3-dimethylacryloyl)-16,16-dimethyl-9-deoxy-$\Delta^9$-$PGD_2$ alcohol,

1-O-(3,3-dimethylacryloyl)-17,20-dimethyl-9-deoxy-$\Delta^9$-$PGD_2$ alcohol,

1-O-(3,3-dimethylacryloyl)-15-(1-butylcyclobutyl)-16,17,18,19,20-pentanor-9-deoxy-$\Delta^9$-$PGD_2$ alcohol,

1-O-(3,3-dimethylacryloyl)-15-cyclopentyl-16,17,18,19,20-pentanor-9-deoxy-$\Delta^9$-$PGD_2$ alcohol,

1-O-(3,3-dimethylacryloyl)-15-(3-propylcyclopentyl)-16,17,18,19,20-pentanor-9-deoxy-$\Delta^9$-$PGD_2$ alcohol,

1-O-(3,3-dimethylacryloyl)-15-(4-butylcyclohexyl)-16,17,18,19,20-pentanor-9-deoxy-$\Delta^9$-$PGD_2$ alcohol,

1-O-(3,3-dimethylacryloyl)-16-phenyl-17,18,19,20-tetranor-9-deoxy-$\Delta^9$-$PGD_2$ alcohol,

1-O-(3,3-dimethylacryloyl)-16-phenoxy-17,18,19,20-tetranor-9-deoxy-$\Delta^9$-$PGD_2$ alcohol,

1-O-(3,3-dimethylacryloyl)-16-(3-chlorophenoxy)-17,18,19,20-tetranor-9-deoxy-$\Delta^9$-$PGD_2$ alcohol,

1-O-(3,3-dimethylacryloyl)-16-(3-trifluoromethylphenoxy)-17,18,19,20-tetranor-9-deoxy-$\Delta^9$-$PGD_2$ alcohol,

1-O-carbamoyl-9-deoxy-$\Delta^9$-$PGD_2$ alcohol,

1-O-carbamoyl-16-methyl-9-deoxy-$\Delta^9$-$PGD_2$ alcohol,

1-O-carbamoyl-16,16-dimethyl-9-deoxy-$\Delta^9$-$PGD_2$ alcohol,

1-O-carbamoyl-17,20-dimethyl-9-deoxy-$\Delta^9$-$PGD_2$ alcohol,

1-O-carbamoyl-15-(1-butylcyclobutyl)-16,17,18,19,20-pentanor-9-deoxy-$\Delta^9$-$PGD_2$ alcohol,

1-O-carbamoyl-15-cyclopentyl-16,17,18,19,20-pentanor-9-deoxy-$\Delta^9$-$PGD_2$ alcohol,

1-O-carbamoyl-15-(3-propylcyclopentyl)-16,17,18,19,20-pentanor-9-deoxy-$\Delta^9$-$PGD_2$ alcohol,

1-O-carbamoyl-15-(4-butylcyclohexyl)-16,17,18,19,20-pentanor-9-deoxy-$\Delta^9$-$PGD_2$ alcohol,

1-O-carbamoyl-16-phenyl-17,18,19,20-tetranor-9-deoxy-$\Delta^9$-$PGD_2$ alcohol,

1-O-carbamoyl-16-phenoxy-17,18,19,20-tetranor-9-deoxy-$\Delta^9$-$PGD_2$ alcohol,

1-O-carbamoyl-16-(3-chlorophenoxy)-17,18,19,20-tetranor-9-deoxy-$\Delta^9$-$PGD_2$ alcohol,

1-O-carbamoyl-16-(3-trifluoromethylphenoxy)-17,18,19,20-tetranor-9-deoxy-$\Delta^9$-$PGD_2$ alcohol,

1-O-(dimethylcarbamoyl)-9-deoxy-$\Delta^9$-$PGD_2$ alcohol,

1-O-(dimethylcarbamoyl)-16-methyl-9-deoxy-$\Delta^9$-$PGD_2$ alcohol,

1-O-(dimethylcarbamoyl)-16,16-dimethyl-9-deoxy-$\Delta^9$-$PGD_2$ alcohol,

1-O-(dimethylcarbamoyl)-17,20-dimethyl-9-deoxy-$\Delta^9$-$PGD_2$ alcohol,

1-O-(dimethylcarbamoyl)-15-(1-butylcyclobutyl)-16,17,18,19,20-pentanor-9-deoxy-$\Delta^9$-$PGD_2$ alcohol,

1-O-(dimethylcarbamoyl)-15-cyclopentyl-16,17,18,19,20-pentanor-9-deoxy-$\Delta^9$-$PGD_2$ alcohol,

1-O-(dimethylcarbamoyl)-15-(3-propylcyclopentyl)-16,17,18,19,20-pentanor-9-deoxy-$\Delta^9$-$PGD_2$ alcohol,

1-O-(dimethylcarbamoyl)-15-(4-butylcyclohexyl)-16,17,18,19,20-pentanor-9-deoxy-$\Delta^9$-$PGD_2$ alcohol,

1-O-(dimethylcarbamoyl)-16-phenyl-17,18,19,20-tetranor-9-deoxy-$\Delta^9$-$PGD_2$ alcohol,

1-O-(dimethylcarbamoyl)-16-phenoxy-17,18,19,20-tetranor-9-deoxy-$\Delta^9$-$PGD_2$ alcohol,

1-O-(dimethylcarbamoyl)-16-(3-chlorophenoxy)-17,18,19,20-tetranor-9-deoxy-$\Delta^9$-$PGD_2$ alcohol,

1-O-(dimethylcarbamoyl)-16-(3-trifluoromethylphenoxy)-17,18,19,20-tetranor-9-deoxy-$\Delta^9$-$PGD_2$ alcohol,

(5Z,14EZ)-11-oxoprosta-5,9,12,14-tetraen-1-ol,

(5Z,14EZ)-11-oxo-16-methylprosta-5,9,12,14-tetraen-1-ol,

(5Z,14EZ)-11-oxo-16,16-dimethylprosta-5,9,12,14-tetraen-1-ol,

(5Z,14EZ)-11-oxo-17,20-dimethylprosta-5,9,12,14-tetraen-1-ol,

(5Z,14EZ)-11-oxo-15-(1-butylcyclobutyl)-16,17,18,19,20-pentanorprosta-5,9,12,14-tetraen-1-ol,

(5Z,14EZ)-11-oxo-15-cyclopentyl-16,17,18,19,20-pentanorprosta-5,9,12,14-tetraen-1-ol,

(5Z,14EZ)-11-oxo-15-(3-propylcyclopentyl)-16,17,18,19,20-pentanorprosta-5,9,12,14-tetraen-1-ol,

(5Z,14EZ)-11-oxo-15-(4-butylcyclohexyl)-16,17,18,19,20-pentanorprosta-5,9,12,14-tetraen-1-ol,

(5Z,14EZ)-11-oxo-16-phenyl-17,18,19,20-tetranorprosta-5,9,12,14-tetraen-1-ol,

(5Z,14EZ)-11-oxo-16-phenoxy-17,18,19,20-tetranorprosta-5,9,12,14-tetraen-1-ol,

(5Z,14EZ)-11-oxo-16-(3-chlorophenoxy)-17,18,19,20-tetranorprosta-5,9,12,14-tetraen-1-ol,

(5Z,14EZ)-11-oxo-16-(3-trifluoromethylphenoxy)-17,18,19,20-tetranorprosta-5,9,12,14-tetraen-1-ol,

(5Z,14EZ)-1-O-acetyl-11-oxoprosta- 5,9,12,14-tetraen-1-ol,

(5Z,14EZ)-1-O-acetyl-11-oxo-16-methylprosta-5,9,12,14-tetraen-1-ol,

(5Z,14EZ)-1-O-acetyl-11-oxo-16,16-dimethylprosta-5,9,12,14-tetraen-1-ol,

(5Z,14EZ)-1-O-acetyl-11-oxo-17,20-dimethylprosta-5,9,12,14-tetraen-1-ol,

(5Z,14EZ)-1-O-acetyl-11-oxo-15-(1-butylcyclobutyl)-16,17,18,19,20-pentanorprosta-5,9,12,14-tetraen-1-ol,

(5Z,14EZ)-1-O-acetyl-11-oxo-15-cyclopentyl-16,17,18,19,20-pentanorprosta-5,9,12,14-tetraen-1-ol,

(5Z,14EZ)-1-O-acetyl-11-oxo-15-(3-propylcyclopentyl)-16,17,18,19,20-pentanorprosta-5,9,12,14-tetraen-1-ol,

(5Z,14EZ)-1-O-acetyl-11-oxo-15-(4-butylcyclohexyl)-16,17,18,19,20-pentanorprosta-5,9,12,14-tetraen-1-ol,

(5Z,14EZ)-1-O-acetyl-11-oxo-16-phenyl-17,18,19,20-tetranorprosta-5,9,12,14-tetraen-1-ol,

(5Z,14EZ)-1-O-acetyl-11-oxo-16-phenoxy-17,18,19,20-tetranorprosta-5,9,12,14-tetraen-1-ol,

(5Z,14EZ)-1-O-acetyl-11-oxo-16-(3-chlorophenoxy)-17,18,19,20-tetranorprosta-5,9,12,14-tetraen-1-ol,

(5Z,14EZ)-1-O-acetyl-11-oxo-16-(3-trifluoromethylphenoxy)-17,18,19,20-tetranorprosta-5,9,12,14-tetraen-1-ol,

(5Z,14EZ)-1-O-(3,3-dimethylacryloyl)-11-oxoprosta-5,9,12,14-tetraen-1-ol,

(5Z,14EZ)-1-O-(3,3-dimethylacryloyl)-11-oxo-16-methylprosta-5,9,12,14-tetraen-1-ol,

(5Z,14EZ)-1-O-(3,3-dimethylacryloyl)-11-oxo-16,16-dimethylprosta-5,9,12,14-tetraen-1-ol,

(5Z,14EZ)-1-O-(3,3-dimethylacryloyl)-11-oxo-17,20-dimethylprosta-5,9,12,14-tetraen-1-ol,

(5Z,14EZ)-1-O-(3,3-dimethylacryloyl)-11-oxo-15-(1-butylcyclobutyl)-16,17,18,19,20-pentanorprosta-5,9,12,14-tetraen-1-ol,

(5Z,14EZ)-1-O-(3,3-dimethylacryloyl)-11- oxo-15-cyclopentyl-16,17,18,19,20-pentanorprosta-5,9,12,14-tetraen-1-ol,

(5Z,14EZ)-1-O-(3,3 dimethylacryloyl)-11-oxo-15-(3-propylcyclopentyl)-16,17,18,19,20-pentanorprosta-5,9,12,14-tetraen-1-ol,

(5Z,14EZ)-1-O-(3,3 dimethylacryloyl)-11-oxo-15-(4-butylcyclohexyl)-16,17,18,19,20-pentanorprosta-5,9,12,14-tetraen-1-ol,

(5Z,14EZ)-1-O-(3,3-dimethylacryloyl)-11-oxo-16-phenyl-17,18,19,20-tetranorprosta-5,9,12,14-tetraen-1-ol,

(5Z,14EZ)-1-O-(3,3-dimethylacryloyl)-11-oxo-16-phenoxy-17,18,19,20-tetranorprosta-5,9,12,14-tetraen-1-ol,

(5Z,14EZ)-1-O-(3,3-dimethylacryloyl)-11-oxo-16-(3-chlorophenoxy)-17,18,19,20-tetranorprosta-5,9,12,14-tetraen-1-ol,

(5Z,14EZ)-1-O-(3,3-dimethylacryloyl)-11-oxo-16-(3-trifluoromethylphenoxy)-17,18,19,20-tetranorprosta-5,9,12,14-tetraen-1-ol,

(5Z,14EZ)-1-O-carbamoyl-11-oxoprosta-5,9,12,14-tetraen-1-ol,

(5Z,14EZ)-1-O-carbamoyl-11-oxo-16-methylprosta-5,9,12,14-pentanorprosta-5,9,12,14-tetraen-1-ol,

(5Z,14EZ)-1-O- carbamoyl-11-oxo-16,16-dimethylprosta- carbamoyl-5,9,12,14-tetraen-1-ol,

(5Z,14EZ)-1-O- carbamoyl-11-oxo-17,20-dimethylprosta-5,9,12,14-tetraen-1-ol,

(5Z,14EZ)-1-O- carbamoyl-11-oxo-15-(1-butylcyclobutyl)-16,17,18,19,20-pentanorprosta-5,9,12,14-tetraen-1-ol,

(5Z,14EZ)-1-O- carbamoyl-11-oxo-15-cyclopentyl-16,17,18,19,20-pentanorprosta-5,9,12,14-tetraen-1-ol,

(5Z,14EZ)-1-O- carbamoyl-11-oxo-15-(3-propylcyclopentyl)-16,17,18,19,20-pentanorprosta-5,9,12,14-tetraen-1-ol,

(5Z,14EZ)-1-O-carbamoyl-11-oxo-15-(4-butylcyclohexyl)-16,17,18,19,20-pentanorprosta-5,9,12,14-tetraen-1-ol,

(5Z,14EZ)-1-O-carbamoyl-11-oxo-16-phenyl-17,18,19,20-tetranorprosta-5,9,12,14-tetraen-1-ol,

(5Z, 14EZ)-1-O-carbamoyl-11-oxo-16-phenoxy-17,18,19,20-tetranorprosta-5,9,12,14-tetraen-1-ol,

(5Z, 14EZ)-1-O-carbamoyl-11-oxo-16-(3-chlorophenoxy)-17,18,19,20-tetranorprosta-5,9,12,14-tetraen-1-ol,

(5Z, 14EZ)-1-O-carbamoyl-11-oxo-16-(3-trifluoromethylphenoxy)-17,18,19,20-tetranorprosta-5,9,12,14-tetraen-1-ol,

(5Z, 14EZ)-1-O-(dimethylcarbamoyl)-11-oxoprosta-5,9,12,14-tetraen-1-ol,

(5Z, 14EZ)-1-O-(dimethylcarbamoyl)-11-oxo-16-methylprosta-5,9,12,14-tetraen-1-ol,

(5Z, 14EZ)-1-O-(dimethylcarbamoyl)-11-oxo-16,16-dimethylprosta-5,9,12,14-tetraen-1-ol,

(5Z, 14EZ)-1-O-(dimethylcarbamoyl)-11-oxo-17,20-dimethylprosta-5,9,12,14-tetraen-1-ol,

(5Z, 14EZ)-1-O-(dimethylcarbamoyl)-11-oxo-15-(1-butylcy-cyclobutyl)-16,17,18,19,20-pentanorprosta-5,9,12,14-tetraen-1-ol,

(5Z, 14EZ)-1-O-(dimethylcarbamoyl)-11-oxo-15-cyclopentyl-16,17,18,19,20-pentanorprosta-5,9,12,14-tetraen-1-ol,

(5Z, 14EZ)-1-O-(dimethylcarbamoyl)-11-oxo-15-(3-propylcyclopentyl)-16,17,18,19,20-pentanorprosta-5,9,12,14-tetraen-1-ol,

(5Z, 14EZ)-1-O-(dimethylcarbamoyl)-11-oxo-15-(4-butylcyclohexyl)-16,17,18,19,20-pentanorprosta-5,9,12,14-tetraen-1-ol,

(5Z,14EZ)-1-O-(dimethylcarbamoyl)-11-oxo-16-phenyl-17,18,19,20-tetranorprosta-5,9,12,14-tetraen-1-ol,

(5Z,14EZ)-1-O-(dimethylcarbamoyl)-11-oxo-16-phenoxy-17,18,19,20-tetranorprosta-5,9,12,14-tetraen-1-ol,

(5Z,14EZ)-1-O-(dimethylcarbamoyl)-11-oxo-16-(3-chlorophenoxy)-17 18,19,20-tetranorprosta-5,9,12,14-tetraen-1-ol,

(5Z,14EZ)-1-O-(dimethylcarbamoyl)-11-oxo-16-(3-trifluoromethylphenoxy)-17,18,19,20-tetranorprosta-5.9,12,14-tetraen-1-ol,

and the corresponding compounds wherein X represents an ethylene group.

The following Reference Examples and Examples illustrate the preparation of compounds of the present invention. In the Reference Examples and Examples 'TLC', 'NMR', 'IR' and 'Mass' represent 'Thin layer chromatography'.'Nuclear magnetic resonance spectrum', 'Infrared absorption spectrum' and 'Mass spectrum', respectively, and in the structural formulae, BMS, THP and MS represent a tert-butyldimethylsilyl group, tetrahydropyran-2-yl group and mesyl group (methanesulfonyl group), respectively. The solvents in parenthesis specified in chromatographic separations show the eluent or the developing solvents. Except when specified otherwise, infrared absorption spectra were recorded by the liquid film method and nuclear resonance spectra were recorded in deuterochloroform ($CDCl_3$) solution.

Reference Example 1

Synthesis of

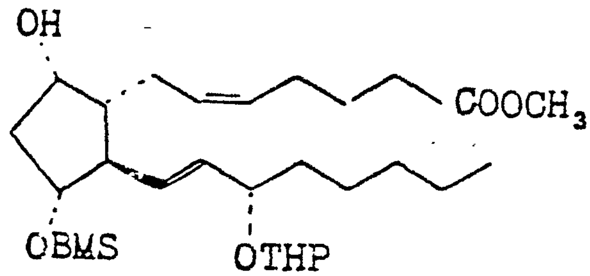

To a solution of 5 g of (5Z,13E)-(9α,11α,15S)-9,11-dihydroxy-15-(tetrahydropyran-2-yloxy) prosta-5,13-dienoic acid methyl ester (the compound described in the Reference Example 1 of the European Patent Publication No. 0098 141) in 55 ml of dry pyridine was added 134 mg of dimethylaminopyridine and 8.33 g of tert-butyldimethylchlorosilane, and the mixture was stirred for one hour at 35°C. After adding 500 ml of diethyl ether, the reaction mixture was washed successively with 1N hydrochloric acid cooled by water, water and a saturated aqueous solution of sodium chloride, and then dried over anhydrous magnesium sulfate and concentrated under reduced pressure. The residue was purified by column chromatography on silica gel (a mixture of n-hexane and ethyl acetate → ethyl acetate only) to give 6.6 g (partially includes the impurities) of the title compound having the following physical characteristics.

TLC (n-hexane : ethyl acetate = 2 : 1) : Rf = 0.74;

NMR : $\delta$ = 5.5 ~ 5.1 (4H, m), 4.7 ~ 4.5 (1H, m),
4.3 ~ 3.2 (5H, m), 3.6 (3H, s),
0.9 (9H, s), 1.0 ~ 0.8 (3H, t);

IR : $\nu$ = 3500, 2930, 1735, 1250, 1015, 975 $cm^{-1}$;

Mass : m/e = 509, 407.

By the same procedure as described in Reference Example 1, the following compounds in table 1 were prepared. The compounds used as starting materials are described respectively in Reference Example 1(a), 1(b), 1(c) and 1(e) of the specification of European Patent Publication No. 0098 141.

table 1

| Reference Example No. | Structural formulae of products | Physical characteristics of products | | |
|---|---|---|---|---|
| | | TLC (Rf) (developing solvents) | NMR (δ) | IR (ν) |
| 1 (a) | 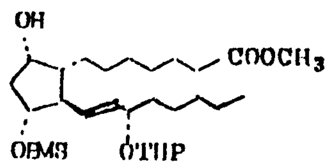 | 0.75 (ethyl acetate : n-hexane = 1 : 2) | 5.5~5.2 (2H, m), 4.6 (1H, m), 3.6 (3H, s), 0.9 (12H, s), 0.1 (6H, s) ( $CCl_4$ solution) | 2,930, 1737 |
| 1 (b) | 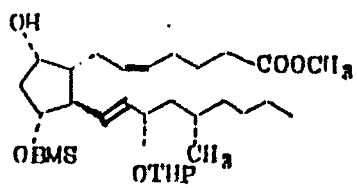 | 0.75 (ethyl acetate : n-hexane = 1 : 2) | 5.5~5.1 (4H, m), 4.6 (1H, m), 3.6 (3H, s), 0.9 (15H, s & m), 0.1 (6H, s) | 3470, 2940, 1740 |
| 1 (c) | 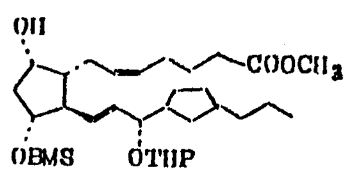 | 0.77 (ethyl acetate : n-hexane = 1 : 2) | 5.7~5.2 (4H, m), 4.7 (1H, m), 3.7 (3H, s), 0.9 (12H, s & t), 0.1 (6H, s) | 3500, 2935, 1740 |
| 1 (d) | 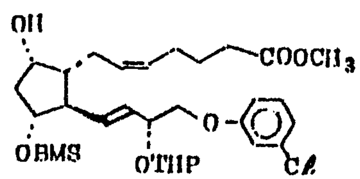 | 0.70 (ethyl acetate : n-hexane = 1 : 2) | 7.3~6.7 (4H, m), 5.7~5.2 (4H, m), 3.7 (3H, s), 0.9 (9H, s), 0.1 (6H, s) | 3500, 2935, 1737, 1600 |

Reference Example 2

Synthesis of

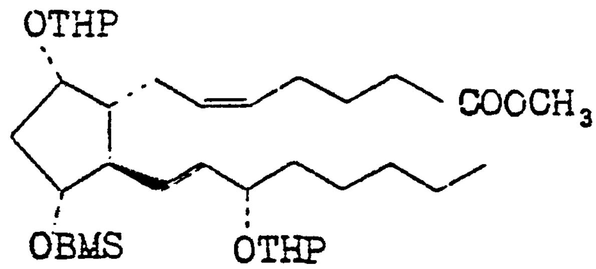

To a solution of 6.6 g of the 9-hydroxy compound (prepared in Reference Example 1) in 56 ml of methylene chloride was added 3.16 ml of 2,3-dihydropyran and 68.5 mg of p-toluenesulfonic acid monohydrate at ambient temperature, and the mixture was stirred for one hour at the same temperature. After adding triethylamine and adjusting to pH9, to the reaction mixture was added 300 ml of diethyl ether, and the mixture was washed successively with water and a saturated aqueous solution of sodium chloride, and then dried over anhydrous magnesium sulfate and concentrated under reduced pressure. The residue was purified by column chromatography on silica gel (n-hexane→ a mixture of n-hexane and ethyl acetate) to give 6.3 g of the title compound having the following physical characteristics.

TLC (n-hexane : ethyl acetate = 5 : 1): Rf = 0.40;

NMR : $\delta$ = 5.6 ~ 5.0 (4H, m), 4.7 ~ 4.4 (2H, m), 4.3 ~ 3.1 (7H, m), 3.6 (3H, s), 0.9 (12H, s and m) ;

IR : $\nu$ = 2920, 2850, 1740, 1450, 1250, 1120, 1020, 985 $cm^{-1}$;

Mass : m/e = 593, 565, 548, 509, 491.

By the same procedure as described in Reference Example 2, the following compounds in table 2 were prepared.

table 2

| Reference Example No. | Starting materials | Structural formulae of products | Physical characteristics of products: TLC (Rf) (developing solvents) | Physical characteristics of products: NMR (δ) | Physical characteristics of products: IR (ν) |
|---|---|---|---|---|---|
| 2 (a) | Reference Example 1 (a) | 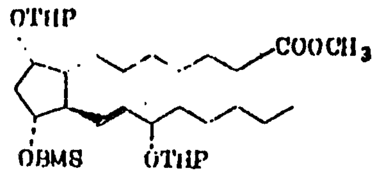 | 0.42 (ethyl acetate : n-hexane = 1 : 5) | 5.6~5.2 (2H, m), 4.7~4.5 (2H, m), 3.6 (3H, s), 0.9 (12H, s), 0.1 (6H, s) | 2930, 1742 |
| 2 (b) | Reference Example 1 (b) | 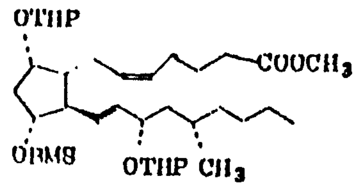 | 0.43 (ethyl acetate : n-hexane = 1 : 5) | 5.6~5.1 (4H, m), 4.7~4.5 (2H, m), 3.6 (3H, s), 0.9 (15H, s & m), 0.1 (6H, s) | 2930, 1740 |
| 2 (c) | Reference Example 1 (c) | 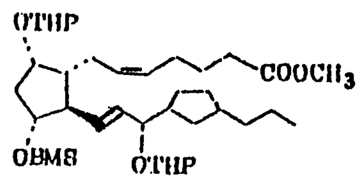 | 0.45 (ethyl acetate : n-hexane = 1 : 5) | 5.6~5.1 (4H, m), 4.7~4.5 (2H, m), 3.7 (3H, s), 0.9 (12H, s & t), 0.1 (6H, s) | 2930, 1740 |
| 2 (d) | Reference Example 1 (d) | 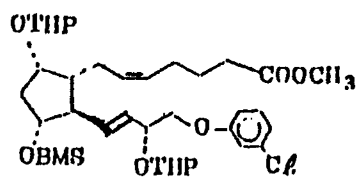 | 0.35 (ethyl acetate : n-hexane = 1 : 5) | 7.3~6.7 (4H, m), 5.6~5.0 (4H, m), 4.7~4.4 (2H, m), 3.7 (3H, s), 0.9 (9H, s), 0.1 (6H, s) | 2935, 1740, 1600 |

Reference Example 3

Synthesis of

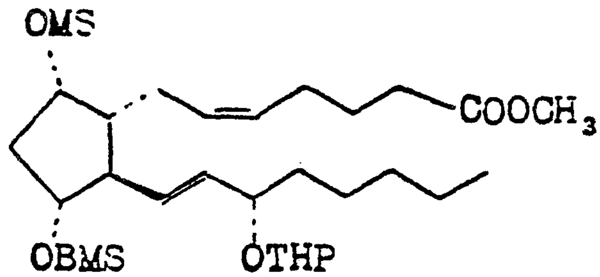

To a solution of 7.13 g of the 9-hydroxy compound (prepared in Reference Example 1) in 60 ml of methylene chloride was added 6.6 ml of triethylamine at -30°C, and further 3.1 ml of mesyl chloride was added dropwise, and the mixture was stirred for 40 minutes at the same temperature and further stirred for 20 minutes at 0°C. To the reaction mixture was added 50 ml of water and 150 ml of methylene chloride, and the methylene chloride layer was separated, washed with a saturated aqueous solution of sodium chloride, and then dried over anhydrous magnesium sulfate and concentrated under reduced pressure. The residue was purified by column chromatography on silica gel (a mixture of n-hexane and ethyl acetate) to give 6.93 g of the title compound having the following physical characteristics.

TLC(n-hexane : ethyl acetate = 3 : 1) : Rf = 0.42;

NMR ($CCl_4$ solution) : $\delta$ = 5.7 ~ 5.2 (4H, m),

4.9 (1H, m), 4.6 (1H, m), 3.6 (3H, s),

2.9 (3H, s), 0.9 (9H, s), 0.1 (6H, s);

IR : $\nu$ = 2940, 1740, 1358, 1173, 912 $cm^{-1}$.

Reference Example 4

Synthesis of 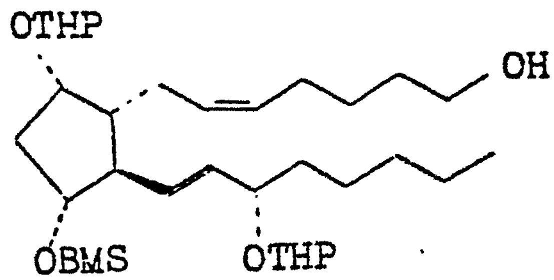

To a solution of 6.3 g of the methyl ester compound (prepared in Reference Example 2) in 50 ml of tetrahydrofuran was added dropwise 12 ml of a 1.76 M toluene solution of diisobutylaluminium hydride at 0°C, and the mixture was stirred for 30 minutes at the same temperature. After adding 5.2 ml of methanol and stirring for 10 minutes at the same temperature, 2 ml of water was added to the reaction mixture, and the mixture was warmed to 35°C stirring vigorously, and further stirred for 30 minutes at the same temperature. The reaction mixture was filtered through a layer of magnesium sulfate, and the filtrate obtained was concentrated under reduced pressure to give 5.64 g of the title compound as crude product having the following physical characteristics.

TLC (n-hexane : ethyl acetate = 5: 1) : Rf = 0.13;

NMR : $\delta$ = 5.6 ~ 5.1 (4H, m), 4.8 ~ 4.4 (2H, m), 4.4 ~ 3.2 (9H, m), 0.9 (12H, s and m);

IR : $\nu$ = 3500, 2930, 2850, 1460, 1255, 1130, 1020, 985 $cm^{-1}$.

By the same procedure as described in Reference Example 4, the following compounds in table 3 were prepared.

table 3

| Reference Example No. | Starting materials | Structural formulae of products | Physical characteristics of products: TLC (Rf) (developing solvents) | NMR (δ) | IR (ν) |
|---|---|---|---|---|---|
| 4 (a) | Reference Example 2 (a) | 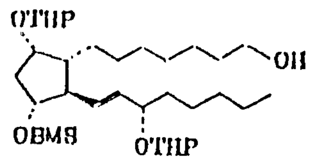 | 0.15 (ethyl acetate : n-hexane = 1 : 5) | 5.6~5.2 (2H, m), 4.8~4.5 (2H, m), 0.9 (12H, s & m), 0.1 (6H, s) | 3500, 2940 |
| 4 (b) | Reference Example 2 (b) | 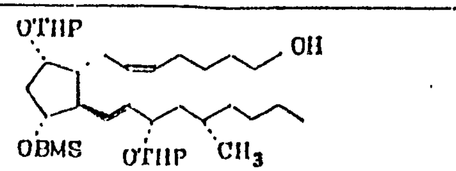 | 0.15 (ethyl acetate : n-hexane = 1 : 5) | 5.6~5.1 (4H, m), 4.8~4.5 (2H, m), 0.9 (15H, s & m), 0.1 (6H, s) | 3500, 2940 |
| 4 (c) | Reference Example 2 (c) | 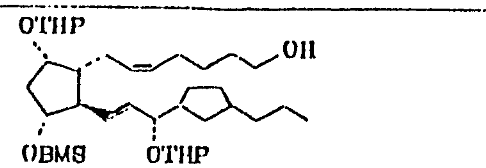 | 0.17 (ethyl acetate : n-hexane = 1 : 5) | 5.6~5.1 (4H, m), 4.8~4.5 (2H, m), 0.9 (12H, s & m), 0.1 (6H, s) | 3480, 2935 |
| 4 (d) | Reference Example 2 (d) | 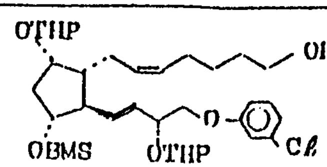 | 0.12 (ethyl acetate : n-hexane = 1 : 5) | 7.3~6.7 (4H, m), 5.6~5.1 (4H, m), 4.8~4.4 (2H, m), 0.9 (9H, s), 0.1 (6H, s) | 3500, 2935, 1600 |
| 4 (e)' | Reference Example 3 | 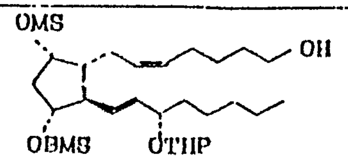 | 0.19 (ethyl acetate : n-hexane = 1 : 2) | 5.6~5.3 (4H, m), 5.1~4.9 (1H, m), 4.8~4.6 (1H, m), 4.2~3.3 (6H, m), 2.97 (3H, s), 0.83 (9H, s) | 3650~3100, 2930, 2850, 1460, 1350, 1255, 1170, 1110, 970 |

Reference Example 5

Synthesis of

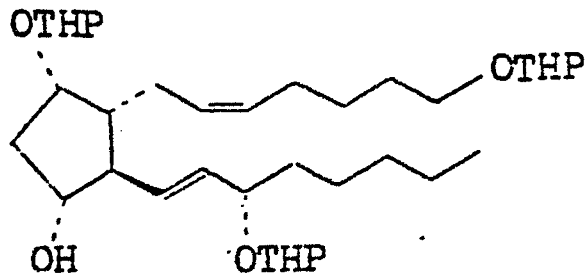

To a solution of 1.72 g of the alcohol compound (prepared in Reference Example 4) in 13 ml of methylene chloride was added 0.49 ml of 2,3-dihydropyran and 5.1 mg of p-toluenesulfonic acid monohydrate, and the mixture was stirred for 5 minutes at 27°C. After adding triethylamine and adjusting to pH8, the reaction mixture was concentrated under reduced pressure. Under an atmosphere of argon, the residue obtained was dissolved in 13 ml of dry tetrahydrofuran, and to the mixture was added 1.4 g of tetrabutylammonium fluoride, and the mixture was stirred for 2 hours at 25°C and further stirred for 30 minutes at 35°C. To the reaction mixture was added 200 ml of diethyl ether and the mixture was washed successively with water and a saturated aqueous solution of sodium chloride, and then dried over anhydrous magnesium sulfate and concentrated under reduced pressure. The residue was purified by column chromatography on silica gel (a mixture of n-hexane and ethyl acetate → ethyl acetate only) to give 1.4 g of the title compound having the following physical characteristics.

TLC (n-hexane : ethyl acetate = 3 : 1) : Rf = 0.11;

NMR : δ = 5.6 ~ 5.1 (4H, m), 4.7 ~ 4.4 (3H, m),
4.4 ~ 3.1 (11H, m);

IR : ν = 3500, 2930, 2860, 1430, 1350, 1200, 1130, 1020, 980 $cm^{-1}$;

Mass : m/e = 490, 406, 388.

By the same procedure as described in Reference Example 5, the following compounds in table 4 were prepared.

table 4

| Reference Example No. | Starting materials | Structural formulae of products | Physical characteristics of products: TLC (Rf) (developing solvents) | NMR (δ) | IR (ν) | Mass (m/e) |
|---|---|---|---|---|---|---|
| 5 (a) | Reference Example 4 (a) | 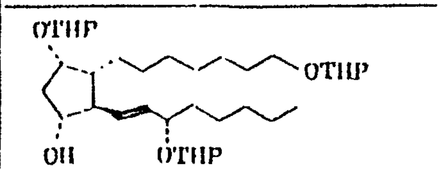 | 0.13 (ethyl acetate : n-hexane = 1 : 3) | 5.6～5.2 (2H, m), 4.7～4.5 (3H, m) | 3500, 2935 | |
| 5 (b) | Reference Example 4 (b) | 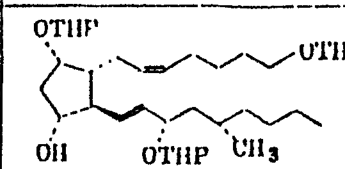 | 0.14 (ethyl acetate : n-hexane = 1 : 3) | 5.6～5.1 (4H, m), 4.7～4.4 (3H, m), 1.0～0.7 (6H, m) | 3500, 2940 | |
| 5 (c) | Reference Example 4 (c) | 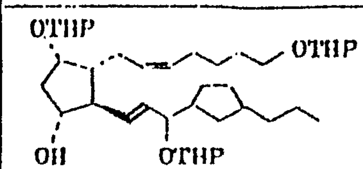 | 0.17 (ethyl acetate : n-hexane = 1 : 3) | 5.6～5.1 (4H, m), 4.7～4.4 (3H, m) | 3550, 2935 | |
| 5 (d) | Reference Example 4 (d) | 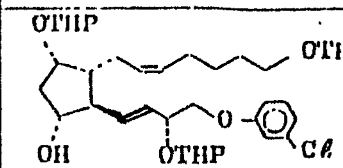 | 0.10 (ethyl acetate : n-hexane = 1 : 3) | 7.3～6.7 (4H, m), 5.6～5.2 (4H, m), 4.8～4.5 (3H, m) | 3500, 2935 1600 | |
| 5 (e) | Reference Example 4 (e) | 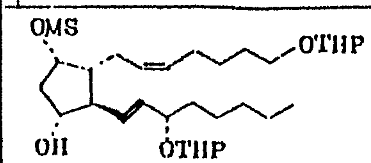 | 0.04 (ethyl acetate : n-hexane = 1 : 2) | 5.7～5.15 (4H, m), 5.15～4.9 (1H, m), 4.8～4.4 (2H, m), 4.2～3.0 (8H, m), 3.0 (3H, s) | 3650～3100 2940, 2860, 1450, 1350, 1170, 1120, 1020, 970 | 370, 304, 286 |

Reference Example 6

Synthesis of

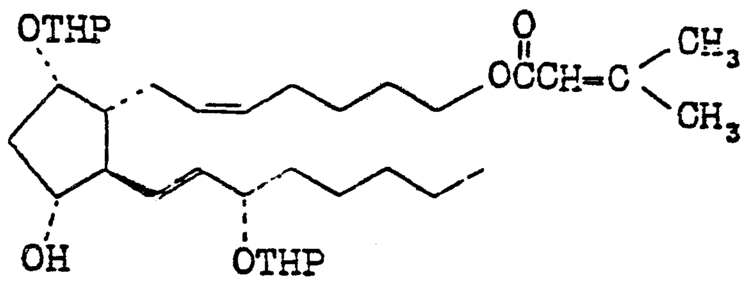

To a solution of 1.92 g of the alcohol compound (prepared in Reference Example 4) in 9 ml of methylene chloride was added 0.73 ml of pyridine and the mixture was cooled to 0°C, 0.5 ml of 3,3-dimethylacryloyl chloride was added dropwise thereto, and the mixture was stirred for 20 minutes at the same temperature. The reaction mixture was poured into 200 ml of ice-water, and the mixture was extracted with 300 ml of diethyl ether. The extract was washed successively with cooled 1N hydrochloric acid, water and a saturated aqueous solution of sodium chloride, and then dried over anhydrous magnesium sulfate and concentrated under reduced pressure. The residue obtained was dissolved in 14.5 ml of dry tetrahydrofuran under an atmosphere of argon, and 2.26 g of tetrabutylammonium fluoride was added to the mixture, and the mixture was stirred for one hour at 27°C and further stirred for 2.5 hours at 35°C. The reaction mixture was poured into 150 ml of ice-water, and the mixture was extracted with 300 ml of ethyl acetate. The extract was washed successively with cooled water

and a saturated aqueous solution of sodium chloride, and then dried over anhydrous magnesium sulfate and concentrated under reduced pressure. The residue was purified by column chromatography on silica gel (methylene chloride → a mixture of ethyl acetate and n-hexane) to give 1.43 g of the title compound having the following physical characteristics.

TLC (ethyl acetate = n-hexane = 1 : 3 ) : Rf = 0.24;

NMR : δ = 5.8 ∼ 5.65 (1H, m), 5.7 ∼ 5.1 (4H, m), 4.8 ∼ 4.5 (2H, m), 4.3 ∼ 3.2 (7H, m), 4.15 (3H, t), 2.18 (3H, s), 1.9 (3H, s);

Mass : m/e = 488, 470, 386.

Reference Example 7

Synthesis of

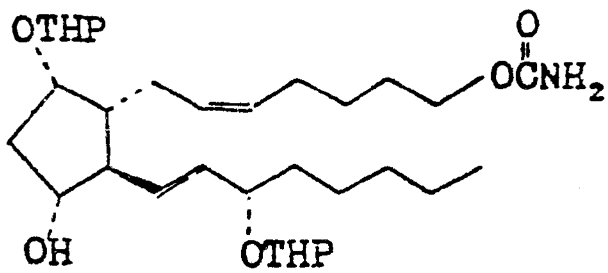

Under an atmosphere of argon, to a solution of 1.937 g of the alcohol compound (prepared in Reference Example 4) in 20 ml of dry diethyl ether was added 0.75 ml of pyridine at ambient temperature and continuously 0.78 ml of phenylchloroformate ( 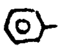 O-COCl) was added dropwise to the mixture at 0°C, and the mixture was stirred for 20 minutes at the same temperature. The reaction mixture was poured into 100 ml of ice-water, and the mixture was extracted with 200 ml of diethyl ether, and the extract was washed successively with water, a saturated aqueous solution of copper sulfate, water and a saturated aqueous solution of sodium chloride, and then dried over anhydrous magnesium sulfate and concentrated under reduced pressure, to obtain a phenylcarbonate compound. Under an atmosphere of argon, to 50 ml of liquid ammonia cooled to -78°C was added the above-mentioned phenylcarbonate compound in 15 ml of diethyl ether at -78°C, and after stirring for 5 hours at -33°C, the reaction mixture was left overnight at ambient temperature and the unreacted liquid ammonia was evaporated. The reaction mixture was diluted

with 300 ml of cooled diethyl ether, and the mixture was washed successively with a 3% aqueous solution of sodium hydroxide (50 ml x 2), cooled water and a saturated aqueous solution of sodium chloride, and then dried over anhydrous magnesium sulfate and concentrated under reduced pressure. The residue obtained was dissolved in 15 ml of dry tetrahydrofuran under an atmosphere of argon, and 2.25 g of tetrabutylammonium fluoride was added to the mixture and stirred for one hour at 27°C and further stirred for 30 minutes at 35°C. The reaction mixture was poured into 150 ml of ice-water, and the mixture was extracted with 300 ml of ethyl acetate. The extract was washed successively with water and a saturated aqueous solution of sodium chloride, and then dried over anhydrous magnesium sulfate and concentrated under reduced pressure. The residue was purified by column chromatography on silica gel (a mixture of n-hexane and ethyl acetate) to give 1.09 of the title compound haveing the following physical characteristics.

TLC (ethyl acetate : n-hexane = 1 : 1 ) : Rf = 0.25;

IR : $\nu$ = 3650 - 3100, 2930, 2850, 1720, 1335, 1120, 1075, 1020, 980 $cm^{-1}$.

By the same procedure as described in Reference Example 7, the following compounds in table 5 were prepared. In Reference Example 7 (a) dimethylamine was used instead of the liquid ammonia in Reference Example 7.

table 5

| Reference Example No. | Starting materials | Structural formulae of products | Physical characteristics of products: TLC(Rf) (developing solvents) | NMR (δ) | IR (ν) | Mass (m/e) |
|---|---|---|---|---|---|---|
| 7 (a) | Reference Example 4 | 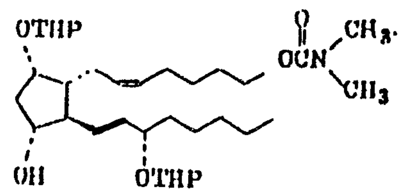 | 0.42 (ethyl acetate : n-hexane = 1 : 1) | | 2940, 1720 | |
| 7 (b) | Reference Example 4 (e) | 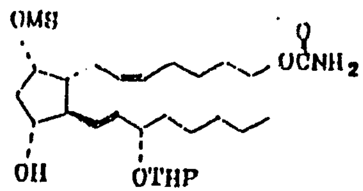 | 0.07 (ethyl acetate : n-hexane = 2 : 1) | 5.85~5.2 (4H, m), 5.2~4.6 (4H, m), 4.03 (2H, t), 4.1~3.2 (5H, m), 3.01 (3H, s) | 3650~3100, 2930, 2860, 1710, 1600, 1330, 1170, 970 | 347, 329 |

Reference Example 8

Synthesis of

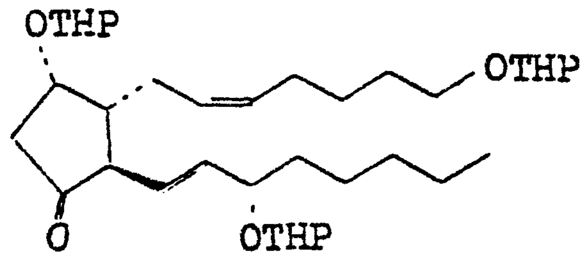

Under an atmosphere of argon, to a solution of 3.81 ml of pyridine in 59 ml of dry methylene chloride was added 2.36 g of chromium trioxide with vigorous stirring and then 12 g of celite, and a further 1.4 g of the 11-hydroxy compound (prepared in Reference Example 5) in 10 ml of methylene chloride was added thereto at 5°C and the mixture was stirred for 10 minutes at the same temperature. The reaction mixture was filtered through a layer of celite, and the filtrate was washed successively with a saturated aqueous solution of copper sulfate, water and a saturated aqueous solution of sodium chloride, and then dried over anhydrous magnesium sulfate and concentrated under reduced pressure to give 1.33 g of the title compound as crude product having the following physical characteristics.

TLC (ethyl acetate = n-hexane = 1 : 3 ) : Rf = 0.29 ;

IR : ν = 2930, 2860, 1740, 1200, 1130, 1075, 980 $cm^{-1}$.

By the same procedure as described in Reference Example 8, the following compounds in table 6 were prepared.

table 6 (1)

| Reference Example No. | Starting materials | Structural formulae of products | Physical characteristics of products: TLC (Rf) (developing solvents) | Physical characteristics of products: IR (/) |
|---|---|---|---|---|
| 8 (a) | Reference Example 5 (a) | 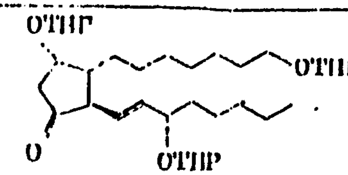 | 0.31 (ethyl acetate : n-hexane = 1 : 3) | 2935, 1742 |
| 8 (b) | Reference Example 5 (b) | 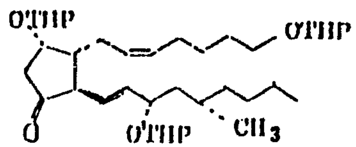 | 0.33 (ethyl acetate : n-hexane = 1 : 3) | 2935, 1740 |
| 8 (c) | Reference Example 5 (c) | 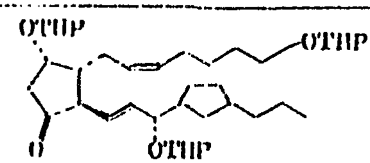 | 0.36 (ethyl acetate : n-hexane = 1 : 3) | 2935, 1740 |
| 8 (d) | Reference Example 5 (d) | 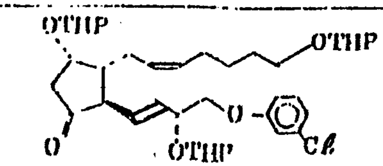 | 0.27 (ethyl acetate : n-hexane = 1 : 3) | 2935, 1740, 1600 |
| 8'(e) | Reference Example 6 | 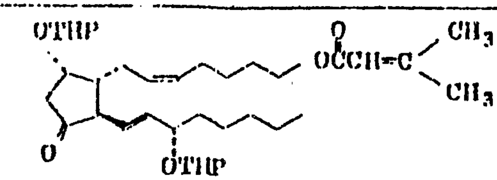 | 0.60 (ethyl acetate : n-hexane = 1 : 2) | 2930, 2850, 1745, 1715, 1650, 1440, 1230, 1145, 1075, 1020, 980 |

table 6 (2)

| Reference Example No. | Starting materials | Structural formulae of products | Physical characteristics of products | | | |
|---|---|---|---|---|---|---|
| | | | TLC(Rf) (developing solvents) | NMR (δ) | IR (ν) | Mass (m/e) |
| 8 (f) | Reference Example 7 | 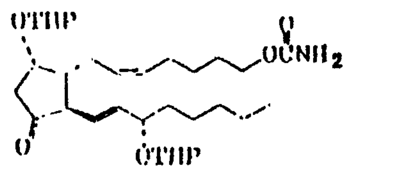 | 0.45 (ethyl acetate : n-hexane = 1 : 1) | 5.72~5.28 (4H, m), 4.9~4.58 (4H, m), 4.49 & 4.27 (1H, t & t), 4.18~3.97 (1H, m), 4.06 (2H, t), 3.97~3.7 (2H, m), 3.6~3.4 (2H, m) | 3650~3100, 2920, 2850, 1730, 1600, 1395, 1330, 1120, 1070, 1015, 980 | 465, 448, 380, 364, 346 |
| 8 (g) | Reference Example 7 (a) | 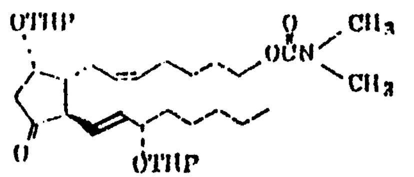 | 0.60 (ethyl acetate : n-hexane = 1 : 1) | 5.72~5.28 (4H, m), 4.6 (2H, m), 4.07 (2H, t), 3.01 (3H, s), 2.95 (3H, s), 0.88 (3H, t) | 2930, 1735, 1605 | |

Reference Example 9

Synthesis of

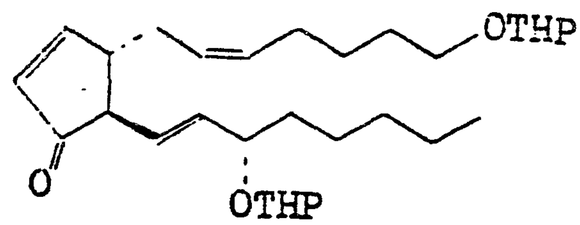

Under an atmosphere of argon, to a solution of 0.5 ml of oxalyl chloride in 11 ml of dry methylene chloride was added dropwise a solution of 0.81 ml of dimethylsulfoxide in 1.1 ml of dry methylene chloride at -50 to -60°C, and the mixture was stirred for 10 minutes at the same temperature. To the mixture was added dropwise a solution of 2.24 g of the 11-hydroxy compound prepared in Reference Example 5 (e) in 3 ml of dry methylene chloride at -50 to 60°C, and the mixture was stirred for 20 minutes at the same temperature. Continuously to the reaction mixture was added dropwise 3.19 ml of triethylamine at -50 to -60°C, and the mixture was stirred for 5 minutes at the same temperature, and further stirred for 30 minutes at ambient temperature. The reaction mixture was poured into 100 ml of ice-water, and the mixture was extracted with 250 ml of diethyl ether, and the extract was washed successively with water and a saturated aqueous solution of sodium chloride, and then dried over anhydrous magnesium sulfate and concentrated under reduced pressure to give 2.25 g of the title compound as crude product having the following physical characteristics.

TLC (ethyl acetate : n-hexane = 1 : 1 ) : Rf = 0.70 ;

NMR : δ = 7.45 (1H, dd), 6.05 (1H, dd).
5.8 ~ 5.0 (4H, m), 4.8 ~ 4.45 (2H, m),
4.3 ~ 3.0 (7H, m);

IR : ν = 2940, 2860, 1710, 1590, 1450, 1350, 1120, 1080, 1030, 980 $cm^{-1}$.

Reference Example 10

Synthesis of

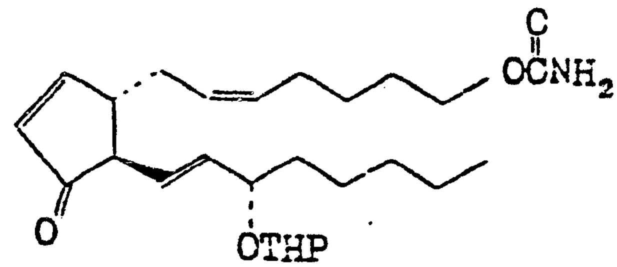

To a solution of 2.61 g of the 11-hydroxy compound (prepared in Reference Example 7 (b)) in 13 ml of acetone was added dropwise 8.9 ml of Jones reagent at -15 to -25°C and the mixture was stirred for 50 minutes at the same temperature. The reaction mixture was poured into 150 ml of ice-water, and the mixture was extracted with 300 ml of ethyl acetate. The extract was washed successively with water and a saturated aqueous solution of sodium chloride, and then dried over anhydrous magnesium sulfate and concentrated under reduced pressure. The residue was purified by column chromatography on silica gel (a mixture of ethyl acetate and n-hexane) to give 785 mg of the title compound having the following physical characteristics.

TLC (ethyl acetate : n-hexane = 3 : 1) : Rf = 0.62;

NMR : $\delta$ = 7.59 (1H, dd), 6.15 (1H, dd),
5.7 ~ 5.2 (4H, m), 5.2 ~ 4.6 (3H, m),
4.05 (2H, t), 4.1 ~ 3.2 (3H, m);

IR : $\nu$ = 3600 ~ 3100, 2930, 2860, 1705, 1600, 1400, 1330, 1070, 970 $cm^{-1}$.

Reference Example 11

Synthesis of

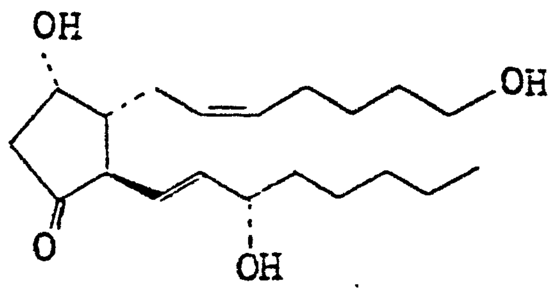

To a solution of 920 ml of 1,9,15—tris—THP compound (prepared in Reference Example 8) in 3.1 ml of tetrahydrofuran was added 15.6 ml of 65% aqueous acetic acid, and the mixture was stirred for 8 minutes at 80°C. To the reaction mixture was added 350 ml of ethyl acetate, and after cooling the mixture was washed successively with water and a saturated aqueous solution of sodium chloride, and then dried over anhydrous magnesium sulfate and concentrated under reduced pressure. The residue was purified by column chromatography on silica gel (a mixture of ethyl acetate and n-hexane ⟶ ethyl acetate only), and further recrystallized from a mixture of diethyl ether and n-hexane to give 115 mg of the title compound having the following physical characteristics.

Melting Point : 45°C;

TLC (chloroform : tetrahydrofuran : acetic acid = 10 : 2 : 1) : Rf = 0.29;

NMR : δ = 5.63 (1H, dd), 5.42 (1H, dd),

5.65 ∼ 5.35 (2H, m), 4.55 ∼ 4.4 (1H, m),

4.09 (1H, dt), 3.66 (2H, t),
2.85 (1H, dd), 2.43 (2H, d);

IR(KBr method) : ν = 3370, 2920, 2850, 1720, 1410, 1310, 1150, 1055, 970 $cm^{-1}$.

Mass : m/e = 338 ($M^+$), 320, 302, 267, 249, 231.

By the same procedure as described in Reference Example 11, the following compound was prepared.

(a)

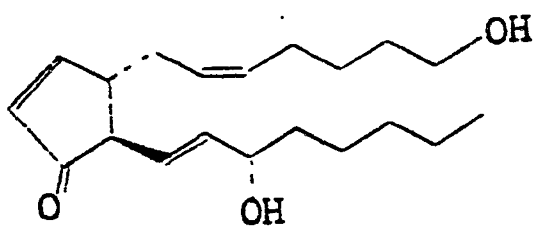

Starting material: the 1,15-bis-THP compound prepared in Reference Example 9;

TLC (chloroform : tetrahydrofuran : acetic acid = 10 : 2 : 1) : Rf = 0.45,

NMR : δ = 7.58 (1H, dd), 6.15 (1H, dd),
5.67 (1H, dd), 5.55 (1H, dd),
5.7 ~ 5.3 (2H, m), 4.09 (1H, dt),
3.64 (2H, t), 2.9 ~ 2.75 (1H, m),
2.66 (1H, dd);

IR : ν = 3650 ~ 3100, 2930, 2860, 1705, 1590, 1460, 1350, 1165, 1050, 970 $cm^{-1}$.

Mass : m/e = 320 ($M^+$), 302, 249, 231.

Example 1

Synthesis of 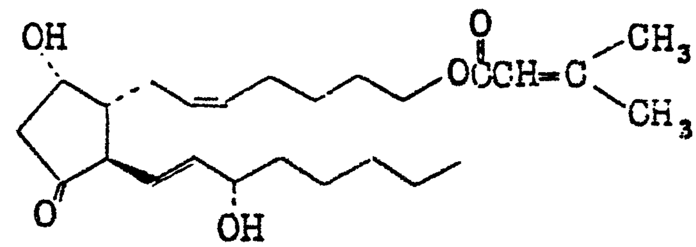

To a solution of 890 mg of the 9,15-bis-THP compound (prepared in Reference Example 8 (e)) in 4.5 ml of tetrahydrofuran was added 15 ml of 65% aqueous acetic acid, and the mixture was stirred for 20 minutes at 80°C. The reaction mixture was poured into 200 ml of ice-water, and the mixture was extracted with 300 ml of diethyl ether. After cooling the extract was washed successively with water and a saturated aqueous solution of sodium chloride, and then dried over anhydrous magnesium sulfate and concentrated under reduced pressure. The residue was purified by column chromatography on silica gel (a mixture of ethyl acetate and n-hexane) to give 280 mg of the title compound having the following physical characteristics.

TLC (ethyl acetate : n-hexane = 1 : 1 ) ; Rf = 0.24;

NMR : $\delta$ = 5.7 ~ 5.65 (1H, m), 5.65 ~ 5.35 (4H, m), 4.52 ~ 4.43 (1H, m), 4.16 ~ 4.0 (3H, m), 2.84 (1H, dd), 2.15 (3H, s), 1.88 (H, s);

IR (KBr method) : $\nu$ = 3650 ~ 3030, 2920, 2850, 1715, 1645, 1440, 1225, 1145, 1075, 970 $cm^{-1}$;

Mass : m/e = 420 ($M^+$), 402, 384, 349, 331.

By the same procedure as described in Example 1, the following compounds in table 7 were prepared.

table 7

| Example No. | Starting materials | Structural formulae of products | Physical characteristics of products: TLC (Rf) (developing solvents) | NMR (δ) | IR (ν) | Mass (m/e) |
|---|---|---|---|---|---|---|
| 1 (a) | Reference Example 8 (f) | 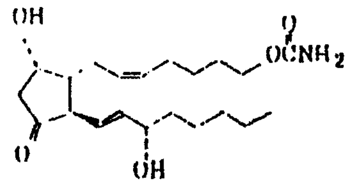 | 0.24 (ehyl acetate : n-hexane = 2 : 1) | 5.63 (1H, dd), 5.43 (1H, dd), 5.7~5.35 (2H, m), 4.7~4.5 (2H, m), 4.55~4.4 (1H, m), 4.06 (2H, t), 4.08 (1H, dt), 2.84 (1H, dd), 2.42 (2H, d) | 3650~3100, 2925, 2850, 1720, 1690, 1605, 1405, 1340, 1075, 970 (KBr method) | 363 ($M^+$), 345, 292, 274 |
| 1 (b) | Reference Example 8 (o) | 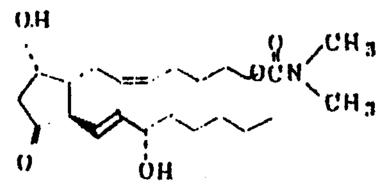 | 0.35 (ethyl acetate : n-hexane = 2 : 1) | 5.70~5.32 (4H, m), 4.55~4.40 (1H, m), 4.07 (2H, t), 3.02 (3H, s), 2.96 (3H, s), 0.89 (3H, t) | 2935, 1725, 1692, 1610 | |
| 1 (c) | Reference Example 10 | 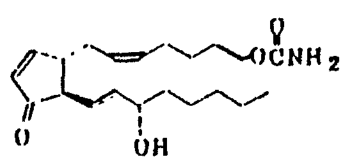 | 0.54 (chloroform : tetrahydrofuran : acetic acid = 10 : 2 : 1) | 7.58 (1H, dd), 6.16 (1H, dd), 5.76~5.28 (4H, m), 4.95~4.5 (2H, m), 4.2~3.96 (1H, m), 4.06 (2H, t), 2.82~2.75 (1H, m), 2.65 (1H, dd) | 3650~3100, 2930, 2860, 1705, 1600, 1405, 1335, 1070, 970 | 363 ($M^+$), 345, 292 |

Example 2

Synthesis of

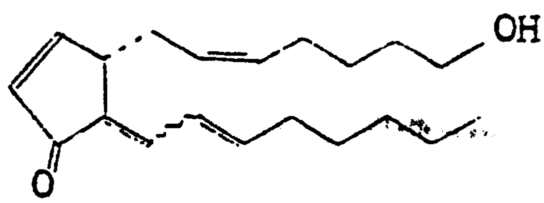

After refluxing a mixture of 380 mg of the 1,9,15-tris-THP compound prepared in Reference Example 8 , 17 ml of tetrahydrofuran and 6.5 ml of 1N aqueous hydrochloric acid for one hour, 250 ml of cooled diethyl ether was added thereto, and after cooling the mixture was washed successively with water and a saturated aqueous solution of sodium chloride, and then dried over anhydrous magnesium sulfate and concentrated under reduced pressure. The residue was purified by column chromatography on silica gel (a mixture of ethyl acetate and n-hexane) to give 110 mg of the title compound having the following physical characteristics.

TLC (chloroform : tetrahydrofuran : acetic acid = 10 : 2 : 1 ) : Rf = 0.67;

NMR : $\delta$ = 7.48 (1H, dd), 6.95 (1H, d), 6.35 (1H, dd), 6.45 $\sim$ 6.15 (2H, m), 5.6 $\sim$ 5.25 (2H, m), 3.63 (2H, t), 3.7 $\sim$ 3.5 (1H, m);

IR : $\nu$ = 3500, 2930, 2850, 1690, 1625, 1580, 1450, 1205, 1070, 1020, 975 $cm^{-1}$;

Mass : m/e = 302 ($M^+$), 231, 190.

By the same procedure as described in Example 2, the following compounds in table 8 were prepared.

table 8 (1)

| Example No. | Starting materials | Structural formulae of products | Physical characteristics of products: TLC(Rf) (developing solvents) | NMR (δ) | IR (ν) | Mass(m/e) |
|---|---|---|---|---|---|---|
| 2 (a) | Reference Example 11 | 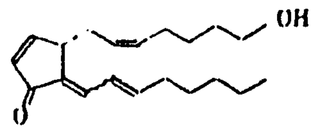 | the same product as Example 2 | the same product as Example 2 | the same product as Example 2 | the same product as Example 2 |
| 2 (b) | Reference Example 8 (a) | 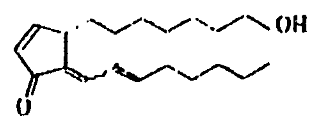 | 0.67 (chloroform : tetrahydrofuran : acetic acid = 10 : 2 : 1) | 7.47 (1H, dd), 6.94 (1H, d), 6.35 (1H, dd), 6.43~6.13 (2H, m) 3.62 (2H, t) | 3500, 2930, 2850, 1692, 1624, 1580 | 304(M⁺) |
| 2 (c) | Reference Example 8 (b) | 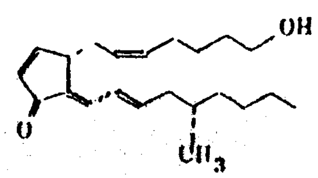 | 0.70 (chloroform : tetrahydrofuran : acetic acid = 10 : 2 : 1) | 7.45 (1H, dd), 6.96 (1H, d), 6.34 (1H, dd), 6.45~6.15 (2H, m), 5.6~5.23 (2H, m), 3.60 (3H, t & m), 1.0~0.7 (6H, m) | 3500, 2935, 1690, 1626, 1583 | 330(M⁺) |
| 2 (d) | Reference Example 8 (c) | 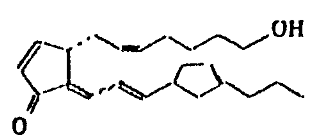 | 0.72 (chloroform : tetrahydrofuran : acetic acid = 10 : 2 : 1) | 7.45 (1H, dd), 6.96 (1H, d), 6.36 (1H, dd), 6.40~6.15 (2H, m), 5.62~5.23 (2H, m), 3.62 (3H, t & m) | 3550, 2935, 1690, 1625, 1585 | 342(M⁺) |

table 8 (2)

| Example No. | Starting materials | Structural formulae of products | Physical characteristics of products: TLC(Rf) (developing solvents) | NMR (δ) | IR (ν) | Mass (m/e) |
|---|---|---|---|---|---|---|
| 2 (e) | Reference Example 8 (d) | 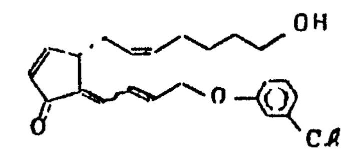 | 0.65 (chloroform : tetrahydrofuran : acetic acid = 10 : 2 : 1) | 7.51~6.70 (6H, m)<br>6.48~6.21 (3H, m)<br>5.61~5.32 (2H, m) | 3500, 2935, 1695, 1630, 1600, 1585 | 374, 372 (M⁺) |
| 2 (f) | Reference Example 8 (e) | 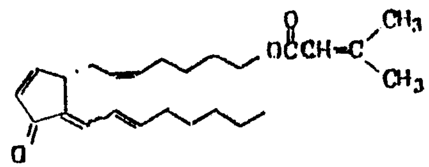 | 0.42 (ethyl acetate : n-hexane = 1 : 5) | 7.45 (1H, dd),<br>6.94 (1H, d),<br>6.33 (1H, dd),<br>6.46~6.12 (2H, m),<br>5.72~5.63 (1H, m),<br>5.56~5.25 (2H, m),<br>4.05 (2H, t),<br>3.63~3.50 (1H, m),<br>2.15 (3H, s),<br>1.88 (3H, s) | 2920, 2850, 1710, 1690, 1630, 1575, 1440, 1225, 1200, 1140, 1070, 970 | 384 (M⁺) 313, 302 |
| 2 (g) | Reference Example 8 (f) | 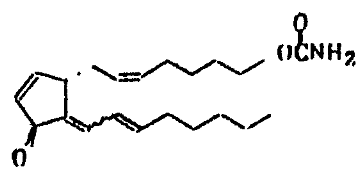 | 0.33 (ethyl acetate : n-hexane = 2 : 3) | 7.46 (1H, dd),<br>6.94 (1H, d),<br>6.34 (1H, dd),<br>6.45~6.1 (2H, m),<br>5.6~5.2 (2H, m),<br>4.8~4.45 (2H, m),<br>4.04 (2H, t),<br>3.7~3.5 (1H, m) | 3650~3100, 2950, 2920, 2850, 1685, 1620, 1425, 1340, 1205, 1070, 975 (KBr method) | 345 (M⁺) 302, 274 |

Example 3

Synthesis of 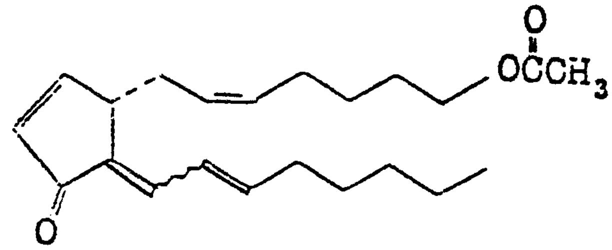

Under an atmosphere of argon, to a solution of 600 mg of the alcohol compound (prepared in Example 2) in 10 ml of dry methylene chloride was added 0.48 ml of pyridine at ambient temperature; after cooling to - 50°C, 0.21 ml of acetyl chloride was added dropwise to the mixture. After stirring for 10 minutes at -60°C, the mixture was warmed to -30°C for 10 minutes, and further stirred for 10 minutes at -30°C. The reaction mixture was poured into 100 ml of ice-water and 250 ml of diethyl ether was added to the mixture, and then the mixture was washed successively with water, a saturated aqueous solution of copper sulfate and a saturated aqueous solution of sodium chloride, and dried over anhydrous magnesium sulfate and concentrated under reduced pressure. The residue was purified by column chromatography on silica gel (a mixture of ethyl acetate and n-hexane) to give 373 mg of the title compound having the following physical characteristics.

TLC (ethyl acetate : n-hexane = 1 : 2 ) : Rf = 0.57;

NMR : $\delta$ = 7.46 (1H, dd), 6.95 (1H, d), 6.35 (1H, dd), 6.45 ~ 6.12 (2H, m), 5.58 ~ 5.24 (2H, m), 4.04 (2H, t), 3.66 ~ 3.50 (1H, m), 2.04 (3H, S);

IR : $\nu$ = 2930, 2860, 1735, 1690, 1630, 1575, 1450, 1365, 1240, 1040, 975 $cm^{-1}$;

Mass :m/e = 344 ($M^+$), 302, 273.

By the same procedure as described in Example 3, the following compounds in table 9 were prepared.

table 9

| Example No. | Starting materials | Structural formulae of products | Physical characteristics of products | | | |
|---|---|---|---|---|---|---|
| | | | TLC(Rf) (developing solvents) | NMR (δ) | IR (ν) | Mass(m/e) |
| 3 (a) | Example 2 (c) | 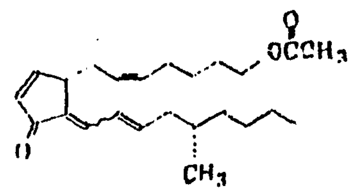 | 0.59 (ethyl acetate : n-hexane = 1 : 2) | 7.46 (1H, dd), 6.96 (1H, d), 6.46 - 6.14 (3H, m) 5.58~5.25 (2H, m) 4.04 (2H, t), 3.58 (1H, m), 2.05 (3H, s), 1.00~0.72 (6H, m) | 2935, 1730, 1692, 1633 | 372(M⁺) |
| 3 (b) | Example 2 (d) | 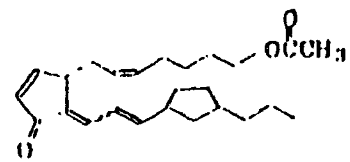 | 0.58 (ethyl acetate : n-hexane =1 : 2) | 7.48 (1H, dd), 6.95 (1H, d), 6.47~6.14 (3H, m), 5.58~5.24 (2H, m), 4.05 (2H, t), 3.60 (1H, m), 2.04 (3H, s) | 2935, 1730, 1695, 1630 | 384(M⁺) |
| 3 (c) | Example 2 (e) | 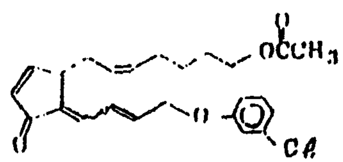 | 0.55 (ethyl acetate : n-hexane = 1 : 2) | 7.51~6.70 (6H, m), 6.48~6.15 (3H, m), 5.60~5.25 (2H, m), 4.04 (2H, t), 3.63 (1H, m), 2.05 (3H, s) | 2935, 1730, 1695, 1632, 1600 | 416, 414(M⁺) |

Example 4

Synthesis of

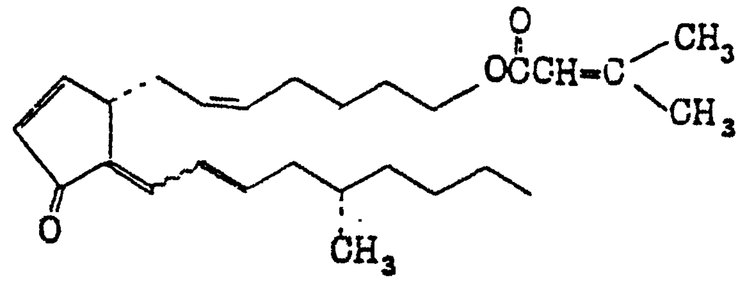

To a solution of 660 mg of the alcohol compound (prepared in Example 2 (c)) in 15 ml of methylene chloride was added 2.5 ml of pyridine, cooling with an ice-bath, 0.89 ml of 3,3-dimethylacryloyl chloride was added dropwise to the mixture, and the mixture was stirred for 30 minutes at the same temperature. To the reaction mixture was added ice and 300 ml of diethyl ether, and the mixture was washed successively with 0.24N aqueous hydrochloric acid and a saturated aqueous solution of sodium chloride, and then dried over anhydrous magnesium sulfate and concentrated under reduced pressure. The residue was purified by column chromatography on silica gel (mixture of ethyl acetate and n-hexane) to give 750 mg of the title compound having the following physical characteristics.

TLC (ethyl acetate : n-hexane = 1 : 5 ) : Rf = 0.44;

NMR : $\delta$ = 7.45 (1H, dd), 6.94 (1H, m),
6.45 ~ 6.13 (3H, m), 5.65 (1H, m),
5.55 ~ 5.26 (2H, m), 4.05 (2H, t),

3.57 (1H, m), 2.16 (3H, S),

2.08 (3H, S), 1.00 ~ 0.72 (6H, m);

IR : ν = 2930, 1712, 1692, 1633 $cm^{-1}$;

Mass : m/e = 4412 ($M^+$).

By the same procedure as described in Example 4, the following compounds in table 10 were prepared.

table 10

| Example No. | Starting materials | Structural formulae of products | Physical characteristics of products: TLC(Rf) (developing solvents) | NMR (δ) | IR (ν) | Mass (m/e) |
|---|---|---|---|---|---|---|
| 4 (a) | Example 2 (d) | 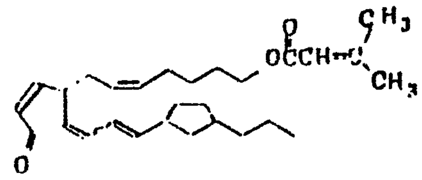 | 0.41 (ethyl acetate : n-hexane = 1 : 5) | 7.49 (1H, dd), 6.97 (1H, d), 6.45~6.13 (3H, m), 5.66 (1H, m), 5.55~5.20 (2H, m), 4.06 (2H, t), 3.56 (1H, m), 2.16 (3H, s), 2.08 (3H, s), 0.89 (3H, t) | 2925, 1711, 1691, 1632, 1575 | 424 ($M^+$) |
| 4 (b) | Example 2 (e) | 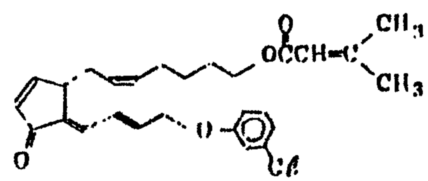 | 0.40 (ethyl acetate : n-hexane = 1 : 5) | 7.52~6.71 (6H, m), 6.50~6.10 (3H, m), 5.65 (1H, m), 5.56~5.27 (2H, m), 4.06 (2H, t), 3.60 (1H, m), 2.17 (3H, s), 2.09 (3H, s) | 2922, 1712, 1690, 1632, 1600, 1575 | 456, 454 ($M^+$) |
| 4 (c) | Reference Example 11 (a) | 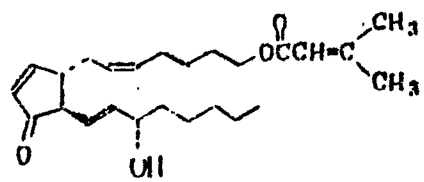 | 0.62 (ethyl acetate : n-hexane = 1 : 1) | 7.58 (1H, dd), 6.16 (1H, dd), 5.70~5.34 (6H, m), 4.08 (2H, m), 2.15 (3H, s), 1.88 (3H, s), 0.87 (3H, t) | 2940, 1718, 1650, 1592, 1224, 1145 | 402 ($M^+$) |

The present invention includes within its scope pharmaceutical compositions which comprise, as active ingredient, a prostaglandin D derivative of general formula I, or cyclodextrin clathrate thereof, in association with a pharmaceutical carrier or coating.

In clinical practice the compounds of the present invention will normally be administered systemically or partially, usually by oral or parenteral (e.g. intravenous, subcutaneous or intramuscular) administration.

The dose to be administered is determined depending upon, for example, age, body weight, symptoms, the desired therapeutic effect, the route of administration, and the duration of the treatment.

In the human adult, the doses per person are generally between 5 mg and 500 mg by oral administration, and between 500 µg and 50 µg by parenteral, preferably intravenous

administration for the prevention or the therapy (including therapy to secure remission) against leukemia, solid cancer, infection or leukocyto-penia and can be administered up to several times per day.

As mentioned above, the doses to be used depend on various factors. Therefore, there may be cases in which doses greater than the ranges specified above, or lower than the ranges specified above, may be used.

In the present invention, solid compositions for oral administration include compressed tablets, pills, dispersible powders and granules. In such solid compositions, one or more of the active compound(s) is, or are, admixed, with at least one inert diluent such as lactose, mannitol, glucose, hydroxypropyl cellulose, starch, polyvinylpyrrolidone or magnesium metasilicate aluminate. The compositions may also comprise, as is normal practice, additional substances other than inert diluents, e.g. lubricating agents such as magnesium stearate, and disintegrating agents, such as cellulose calcium gluconate. The tablets or pills may, if desired, be made into enteric film-coated or gastric film-coated, hydroxypropylcellulose-coated, hydroxypropylmethylcellulose phthalate-coated white sugar-coated or gelatine-coated tablets or pills; two or more layers may be used. Compositions in the form of capsules of absorbable materials such as gelatin can also be used.

Liquid compositions for oral administration include pharmaceutically acceptable emulsions, solutions, suspensions, syrups and elixirs containing inert diluents

commonly used in the art such as distilled water or ethanol. Besides inert diluents such compositions may also comprise adjuvants such as wetting and suspending agents, and sweetening, flavouring, perfuming and preserving agents.

Other compositions for oral administration include spray compositions which may be prepared by known methods and which comprise one or more active substances.

Preparations according to the invention for parenteral administration include sterile aqueous or non-aqueous solutions, suspensions or emulsions. Examples of aqueous solvents or suspending media are distilled water for injection and physiological salt solution. Examples of non-aqueous solvents or suspending media are propylene glycol, polyethylene glycol, vegetable oils such as olive oil, alcohols such as ethanol, and Polysorbate 80 (registered Trade Mark). These compositions may also include adjuvants such as preserving, wetting, emulsifying and dispersing agents. They may be sterilized, for example, by filtration through a bacteria-retaining filter, by incorporation of sterilizing agents in the compositions or by irradiation. They may also be manufactured in the form of sterile solid compositions which can be dissolved in sterile water or some other sterile injectable medium immediately before use.

Other compositions include, for parenteral administration, liquids for external use, and endermic liniments such as ointments; suppositories for rectal administration; and pessaries for vaginal administration. Such compositions are prepared by known methods.

The following Examples illustrate pharmaceutical compositions according to the invention.

Example 5

A solution of one gram of (5Z,14EZ)-11-oxoprosta-5,9,12,14-tetraen-1-ol in 5 ml of ethanol was well mixed with 5 g microcrystalline cellulose. After drying the mixture sufficient, 100 mg of magnesium stearate, 20 mg of silicon dioxide, 10 mg of talc, 200 mg cellulose calcium gluconate (CCG) and further microcrystalline cellulose to produce 10 g of mixture were added. After mixing well to ensure homogeneity, the mixture was punched out in conventional manner to obtain 100 tablets each containing 10 mg of the active ingredient.

Example 6

The α-cyclodextrin and β-cyclodextrin clathrate of (5Z,14EZ)-11-oxoprosta-5,9,12,14-tetraen-1-ol (prepared by dissolving 2.4 g of β-cyclodextrin and 1 g of α-cyclodextrin in 300 ml of water, adding thereto 100 mg of (5Z,14EZ)-11-oxoprosta-5,9,12,14-tetraen-1-ol, stirring well and then concentrating under reduced pressure) was dissolved in 150 ml of distilled water for injection. The solution was sterilized by filtration in conventional manner, placed in 1.5 ml portions in 5 ml ampoules and freeze-dried to obtain 100 ampoules of solid composition for injection each containing 1 mg of the active ingredient.

CLAIMS

1. A prostaglandin D derivative of the formula:

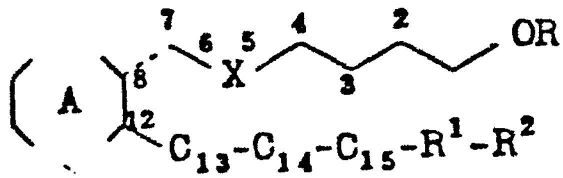

(I)

[wherein [A] represents a group of the formula:

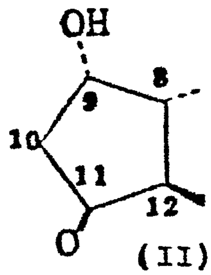

(II),

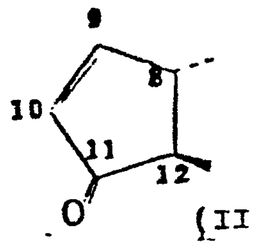

(III) or

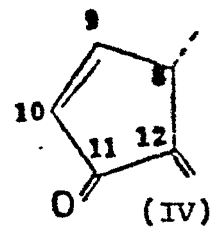

(IV)

X represents an ethylene group or a cis-vinylene group, $C_{13}$-$C_{14}$-$C_{15}$ represents:

(i) a group of the formula:

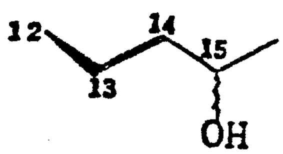

(V)

when [A] represents a group of the formula (II) or (III), or (ii) a group of the formula:

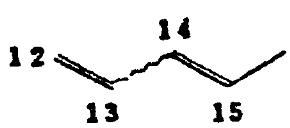

(VI)

when [A] represents a group of the formula (IV), R represents

(i) a group of the formula:

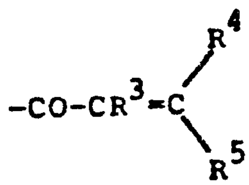 or 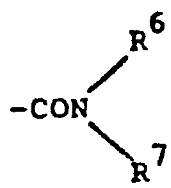

(wherein $R^3$, $R^4$, $R^5$, $R^6$ and $R^7$ which may be the same or different each represents a hydrogen atom or a straight-chain or branched-chain alkyl group of 1-4 carbon atoms) when [A] represents a group of the formula (II) or (III), or
(ii) a hydrogen atom or a group of the formula:

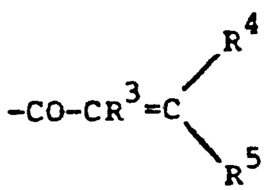 , 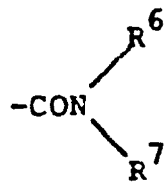 or $-COR^8$

(wherein $R^8$ represents a straight-chain or branched-chain alkyl group of 1-4 carbon atoms, and the other symbols are as hereinbefore defined), when [A] represents a group of the formula (IV), $R^1$ represents a single bond or a straight-chain or branched-chain alkylene group of 1-5 carbon atoms, $R^2$ represents a straight-chain or branched-chain alkyl group of 1-8 carbon atoms, a cycloalkyl group of 4-7 carbon atoms either unsubstituted or substituted by at least one straight-chain or branched-chain alkyl group of 1-8 carbon atoms, or a phenyl or phenoxy group either unsubstituted or substituted by at least one halogen atom, trifluoromethyl group or straight-chain or branched-chain alkyl group of 1-4

carbon atoms, the double bond between $C_9$-$C_{10}$ in the formulae (III) and (IV) being Z, the double bond between $C_{13}$-$C_{14}$ in the formula (V) being E and the double bonds between $C_{12}$-$C_{13}$ and betwen $C_{14}$-$C_{15}$ in the formula (VI) which may be in the same or different configuration, being E, Z or a mixture thereof, with the proviso that when $R^1$ represents a single bond, $R^2$ does not represent a substituted or unsubstituted phenoxy group] or a cyclodextrin clathrate thereof.

2. A compound according to claim 1 wherein the grouping $R^1$-$R^2$ represents pentyl, 2-methylhexyl, 3-propylcyclopentyl or 3-chlorophenoxymethyl.

3. A compound according to claim 1 or 2 wherein $R^3$, $R^4$ and $R^5$ each represents a hydrogen atom or a methyl or ethyl group, or $R^6$ and $R^7$ each represents a hydrogen atom or a methyl or ethyl group, or $R^8$ represents a methyl or ethyl group.

4. A compound according to claim 1, 2 or 3 wherein R represents hydrogen, 3,3-dimethylacryloyl, carbamoyl, dimethylcarbamoyl or acetyl.

5. A compound according to any one of claims 1 to 4 wherein X represents <u>cis</u>-vinylene.

6. A compound according to any one of claims 1 to 5 wherein, when $C_{13}$-$C_{14}$-$C_{15}$ represents a group of the formula (V), the hydroxy group attached to the 15-position carbon atom is in $\alpha$-configuration.

7. A compound according to claim 1, which is 1-O-(3,3-dimethylacryloyl-$PGD_2$ alcohol, 1-O-carbamoyl-$PGD_2$ alcohol, 1-O-(dimethylcarbamoyl)-$PGD_2$ alcohol, 1-O-(3,3-dimethylacryloyl)-9-deoxy-$\Delta^9$-$PGD_2$ alcohol, 1-O-carbamoyl-9-deoxy-$\Delta^9$-$PGD_2$ alcohol, (5Z,14EZ)-11-oxoprosta-5,9,12,14-tetraen-1-ol, (14EZ)-11-oxoprosta-9,12,14-trien-1-ol, (5Z,14EZ)-(17S)-11-oxo-17,20-dimethylprosta-5,9,12,14,-tetraen-1-ol, (5Z,14EZ)-11-oxo-15-(3-propylcyclopentyl)-16,17,18,19,20-pentanorprosta-5,9,12,14-tetraen-1-ol,

(5Z,14EZ)-11-oxo-16-(3-chlorophenoxy)-17,18,19,20-tetranorprosta-5,9,12,14-tetraen-1-ol, (5Z,14EZ)-1-O-(3,3-dimethylacryloyl)-11-oxoprosta-5,9,12,14-tetraen-1-ol, (5Z,14EZ)-(17S)-1-O-(3,3-dimethylacryloyl)-11-oxo-17,20-dimethylprosta-5,9,12,14-tetraen-1-ol, (5Z,14EZ)-1-O-(3,3-dimethylacryloyl)-11-oxo-15-(3-propylcyclopentyl)-16,17,18,19,20-pentanorprosta-5,9,12,14-tetraen-1-ol, (5Z,14EZ)-1-O-(3,3-dimethylacryloyl)-11-oxo-16-(3-chlorophenoxy)-17,18,19,20-tetranorprosta-5,9,12,14-tetraen-1-ol, (5Z,14EZ)-1-O-carbamoyl-11-oxoprosta-5,9,12,14-tetraen-1-ol, (5Z,14EZ)-1-O-acetyl-11-oxoprosta-5,9,12,14-tetraen-1-ol, (5Z,14EZ)-(17S)-1-O-acetyl-11-oxo-17,20-dimethylprosta-5,9,12,14-tetraen-1-ol, (5Z,14EZ)-1-O-acetyl-11-oxo-15-(3-propylcyclopentyl)-16,17,18,19,20-pentanorprosta-5,9,12,14-tetraen-1-ol, or (5Z,14EZ)-1-O-acetyl-11-oxo-16-(3-chlorophenoxy)-17,18,19,20-tetranorprosta-5,9,12,14-tetraen-1-ol.

8. A process for the preparation of prostaglandin D derivatives as claimed in claim 1 which comprises:

(A) when the prostaglandin D derivatives are of the general formula:

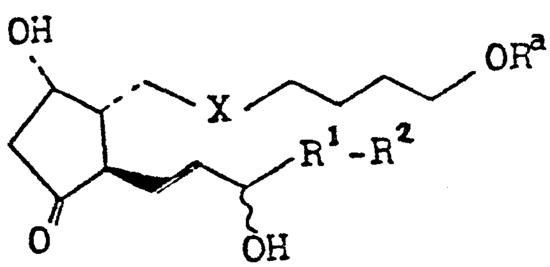

Ia

or

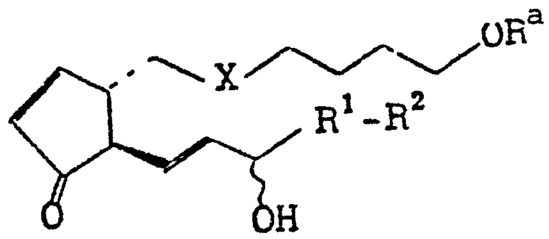

Ib

[wherein $R^a$ represents a group

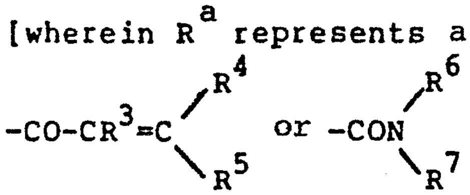

(wherein all of the symbols are as defined in claim 1) and the other symbols are as defined in claim 1], the hydrolysis under acidic conditions of compounds of the general formula

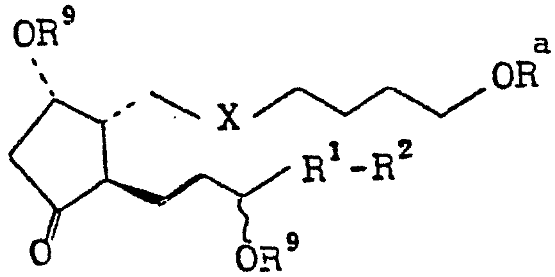

IXa

or

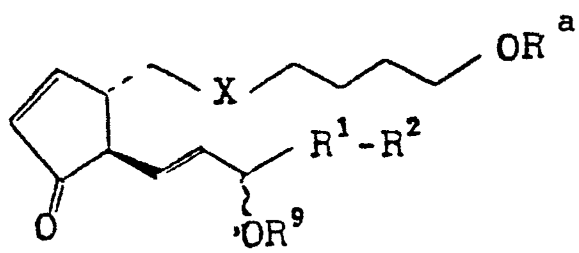

Xa

[wherein $R^9$ represents a tetrahydropyran-2-yl or tetrahydrofuran-2-yl group, either unsubstituted or substituted by at least one alkyl group or a 1-ethoxyethyl

group, $R^a$ is a hereinbefore defined and the other symbols are as defined in claim 1_7 to convert to hydroxy groups the groups $OR^9$;

(F) when the prostaglandin D derivatives are of the formula:

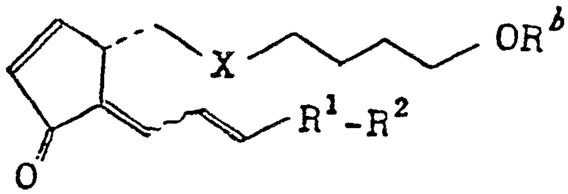

Ic

[wherein $R^b$ represents a hydrogen atom or a group 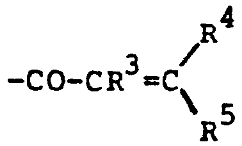 , 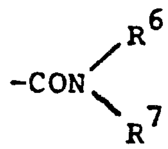 or $-COR^8$ (wherein all of the symbols are as defined in claim 1) and the other symbols are as defined in claim 1_7, reaction with an aqueous acid at the reflux temperature, of a compound of the general formula:

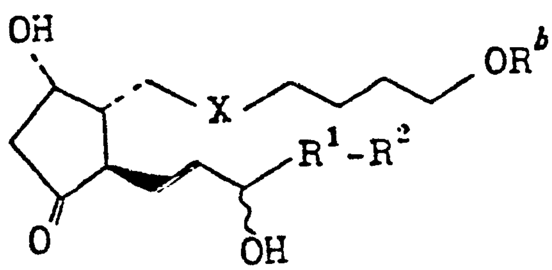

VII

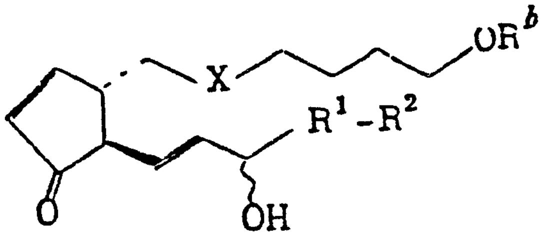

VIII

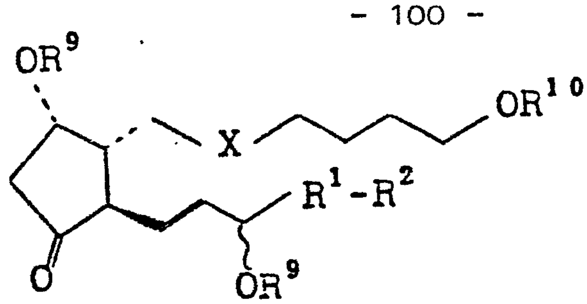

IX

or

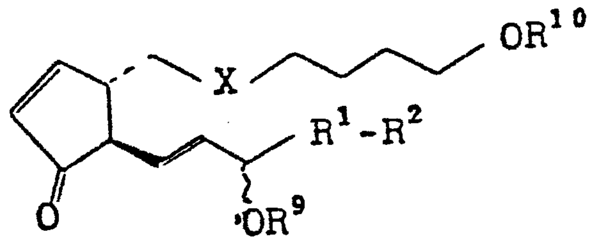

X

/‾ wherein $R^{10}$ represents a group $R^9$ as hereinbefore defined or a group 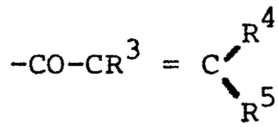 , 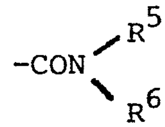 or $-COR^8$ (wherein all of the symbols are as defined in claim 1), $R^b$ is as hereinbefore defined and the other symbols are as defined in claim 1_7;

(C) when the prostaglandin D derivatives are of the general formula:

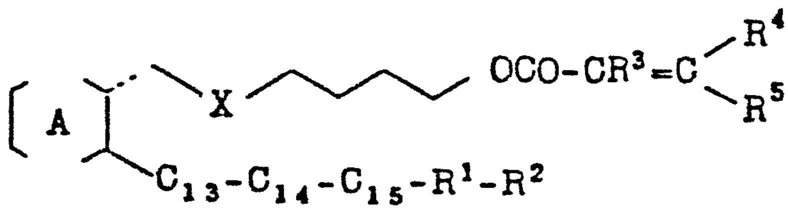

(Id)

(wherein all of the symbols are as defined in claim 1) the reaction of a compound of the general formula:

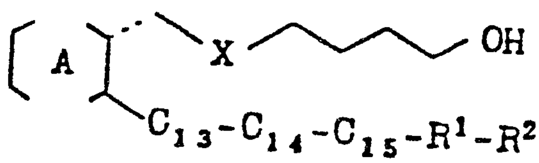

(XI)

(wherein all of the symbols are as defined in claim 1) with an acid halide of the general formula:

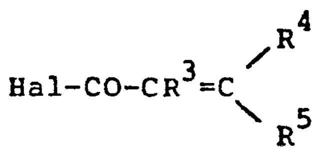

(XII)

(wherein Hal represents a chlorine, bromine or iodine atom and the other symbols are as defined in claim 1) to convert the hydroxy group attached to the 1-position to a group

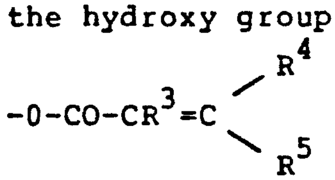

; or

(D) when the prostaglandin D derivatives are of the general formula:

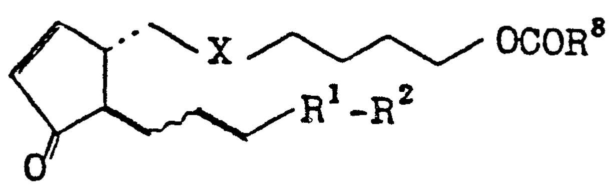

(Ie)

(wherein all of the symbols are as defined in claim 1), the reaction of a compound of the general formula:

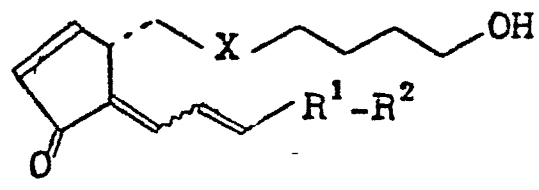

(XIα)

(wherein all of the symbols are as defined in claim 1) with an acid halide of the general formula:

$$Hal-CO-R^8 \qquad (XIII)$$

(wherein Hal is as hereinbefore defined and $R^8$ is as defined in claim 1) to convert the hydroxy group attached to the 1-position to a group $-OCOR^8$;

optionally followed by the step of converting a prostaglandin D derivative obtained into a cyclodextrin clathrate thereof.

9. A prostaglandin D derivative according to claim 1, or cyclodextrin clathrate thereof, when prepared by a process claimed in claim 8.

10. A pharmaceutical composition which comprises, as active ingredient, a prostaglandin D derivative of formula I depicted in claim 1, wherein the various symbols are as defined in claim 1, or a cyclodextrin clathrate thereof in association with a pharmaceutically acceptable diluent or carrier.

11. A prostaglandin derivative as claimed in claim 1 or a cyclodextrin clathrate thereof for use in therapy.

12. A compound of general formula IX or X wherein $R^9$ and $R^{10}$ are as defined in claim 8 and the other symbols are as defined in claim 1.

CLAIMS FOR AUSTRIA

CLAIMS

1. A process for the preparation of a prostaglandin D derivative of the formula:

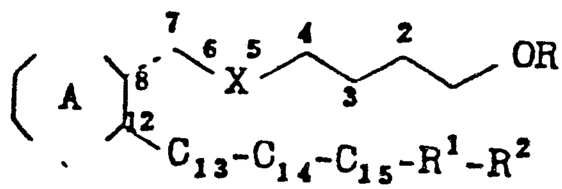

(I)

[wherein [A] represents a group of the formula:

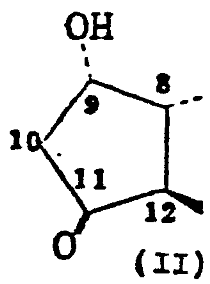

(II),

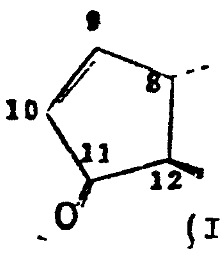

(III) or

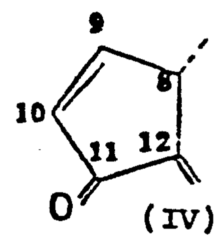

(IV)

X represents an ethylene group or a cis-vinylene group,

$C_{13}-C_{14}-C_{15}$ represents:

(i) a group of the formula:

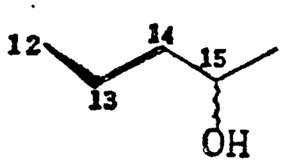

when [A] represents a group of the formula (II) or (III), or

(ii) a group of the formula:

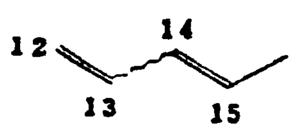

when [A] represents a group of the formula (IV), R represents
(i) a group of the formula:

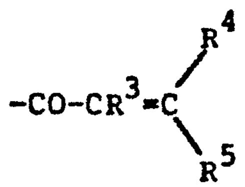 or 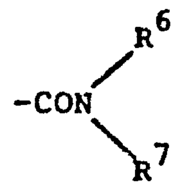

(wherein $R^3$, $R^4$, $R^5$, $R^6$ and $R^7$ which may be the same or different each represents a hydrogen atom or a straight-chain or branched-chain alkyl group of 1-4 carbon atoms) when [A] represents a group of the formula (II) or (III), or
(ii) a hydrogen atom or a group of the formula:

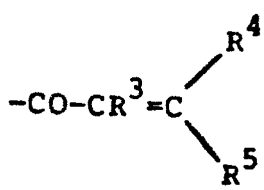 , 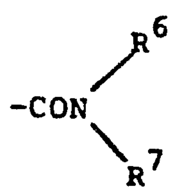 or $-COR^8$

(wherein $R^8$ represents a straight-chain or branched-chain alkyl group of 1-4 carbon atoms, and the other symbols are as hereinbefore defined), when [A] represents a group of the formula (IV), $R^1$ represents a single bond or a straight-chain or branched-chain alkylene group of 1-5 carbon atoms, $R^2$ represents a straight-chain or branched-chain alkyl group of 1-8 carbon atoms, a cycloalkyl group of 4-7 carbon atoms either unsubstituted or substituted by at least one straight-chain or branched-chain alkyl group of 1-8 carbon atoms, or a phenyl or phenoxy group either unsubstituted or substituted by at least one halogen atom, trifluoromethyl group or straight-chain or branched-chain alkyl group of 1-4

carbon atoms, the double bond between $C_9$-$C_{10}$ in the formulae (III) and (IV) being Z, the double bond between $C_{13}$-$C_{14}$ in the formula (V) being E and the double bonds between $C_{12}$-$C_{13}$ and betwen $C_{14}$-$C_{15}$ in the formula (VI) which may be in the same or different configuration, being E, Z or a mixture thereof, with the proviso that when $R^1$ represents a single bond, $R^2$ does not represent a substituted or unsubstituted phenoxy group] characterised in that the process comprises:

(A) when the prostaglandin D derivatives are of the general formula:

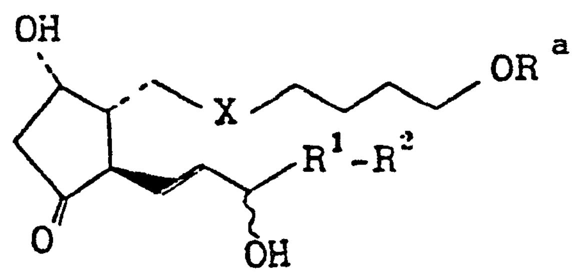

Ia

or

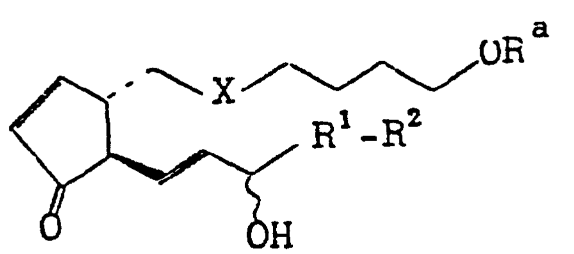

Ib

[wherein $R^a$ represents a group

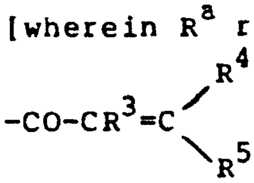 or 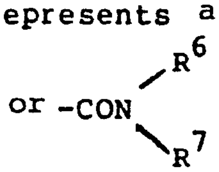 (wherein all of the symbols are as hereinbefore defined) and the other symbols

are as hereinbefore defined], the hydrolysis under acidic conditions of compounds of the general formula

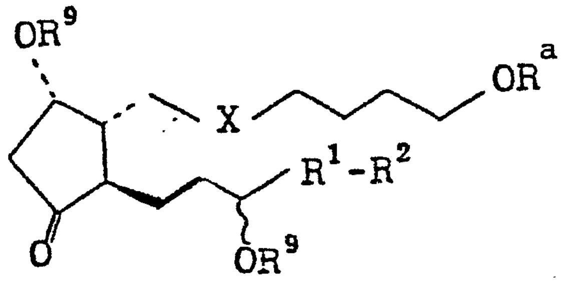

IX a

or

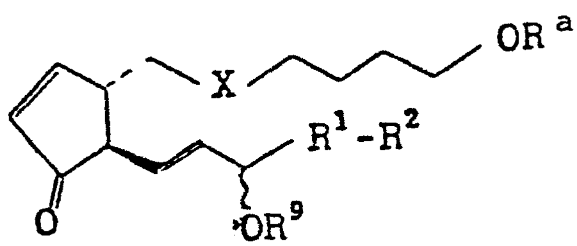

X a

[wherein $R^9$ represents a tetrahydropyran-2-yl or tetrahydrofuran-2-yl group, either unsubstituted or substituted by at least one alkyl group or a 1-ethoxyethyl group,

and the other symbols are as hereinbefore defined] to convert to hydroxy groups the groups $OR^9$;

(B) when the prostaglandin D derivatives are of the formula:

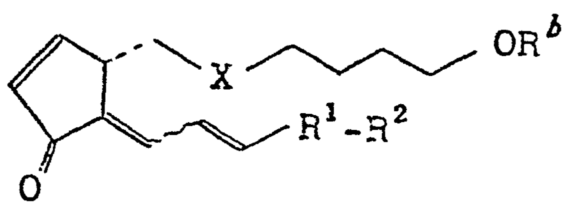

Ic

[wherein $R^b$ represents a hydrogen atom or a group 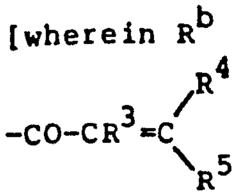 , 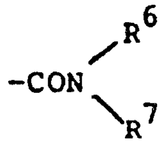 or $-COR^8$ (wherein all of the symbols are as hereinbefore defined) and the other symbols are as hereinbefore defined_7, reaction with an aqueous acid at the reflux temperature, of a compound of the general formula:

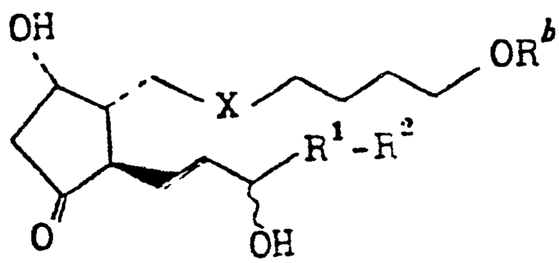

VII

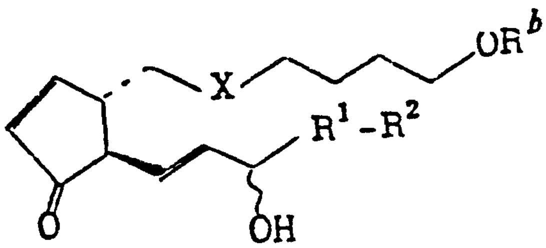

VIII

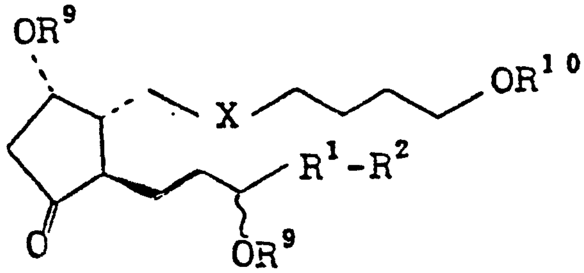

IX

or

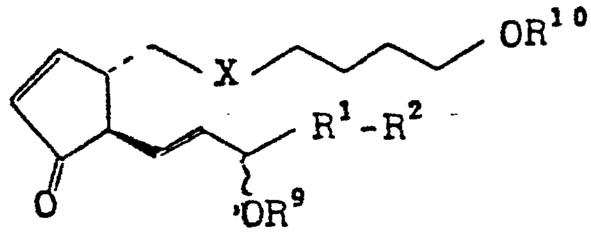

X

⁄‾wherein $R^{10}$ represents a group $R^9$ as hereinbefore defined or a group 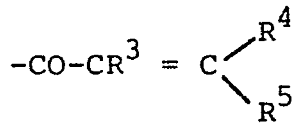 , 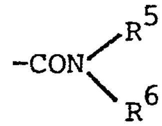 or $-COR^8$ (wherein all of the symbols are as hereinbefore defined) and the other symbols are as hereinbefore defined_7;

(C) when the prostaglandin D derivatives are of the general formula:

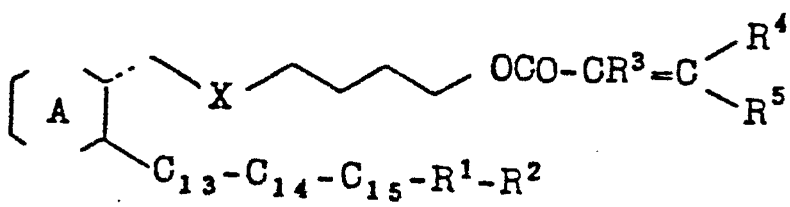

(Id)

(wherein all of the symbols are as hereinbefore defined) the reaction of a compound of the general formula:

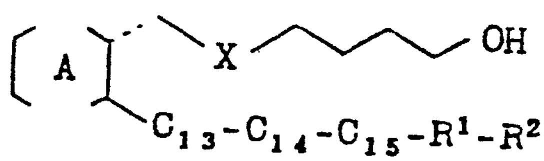

(XI)

(wherein all of the symbols are as hereinbefore defined) with an acid halide of the general formula:

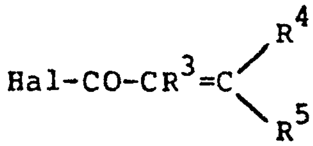

(XII)

(wherein Hal represents a chlorine, bromine or iodine atom and the other symbols are as hereinbefore defined) to convert the hydroxy group attached to the 1-position to a group

$-O-CO-CR^3=C\begin{matrix}R^4\\R^5\end{matrix}$ ; or

(D) when the prostaglandin D derivatives are of the general formula:

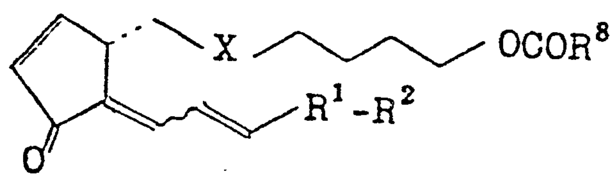

(Ie)

(wherein all of the symbols are as hereinbefore defined), the reaction of a compound of the general formula:

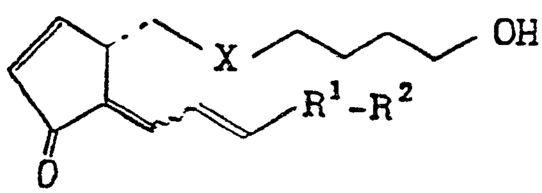

(XIa)

(wherein all of the symbols are as hereinbefore defined) with an acid halide of the general formula:

$$Hal-CO-R^8 \qquad (XIII)$$

(wherein Hal is as hereinbefore defined and $R^8$ is as hereinbefore defined) to convert the hydroxy group attached to the 1-position to a group $-OCOR^8$;

optionally followed by the step of converting a prostaglandin D derivative obtained into a cyclodextrin clathrate thereof.

2. A process according to claim 1 characterised in that the symbol $R^9$ and, when $R^{10}$ represents a group $R^9$, the symbol $R^{10}$ represents a tetrahydropyran-2-yl group.

3. A process according to claim 1 or 2 characterised in that the hydrolysis under acidic conditions in process (A) is carried out using a mixture of dilute hydrochloric acid and tetrahydrofuran, a mixture of dilute hydrochloric acid and methanol, a mixture of acetic acid, water and tetrahydrofuran, a mixture of phosphoric acid, water and tetrahydrofuran, or a mixture of p-toluenesulfonic acid and absolute methanol.

4. A process according to claim 1 characterised in that process (B) is carried out in tetrahydrofuran using 1N hydrochloric acid at the reflux temperature of the reaction mixture.

5. A process according to claim 1 characterised in that process (C) or (D) is carried out at $0^oC$ to $-20^oC$ in an inert organic solvent in the presence of pyridine or triethylamine.

European Patent Office

# EUROPEAN SEARCH REPORT

0171441
Application number

EP 84 10 9568

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,Y | US-A-4 203 924 (N.A. NELSON) * Whole document * & JP - A - 52 39650 | 1-12 | C 07 C 177/00<br>C 08 B 37/16<br>A 61 K 31/557 |
| D,Y | US-A-4 032 576 (N.A. NELSON) * Column 9, line 30 - column 14, line 65 * & JP - A - 52 89650 | 1-12 | |
| Y | GB-A-2 009 162 (AMERICAN CYANAMID) * Page 1, line 44 - page 3, line 31; claims 1,2 * | 1-12 | |
| Y | GB-A-2 002 349 (I.C.I.) * Claims * | 1-12 | |
| | ----- | | **TECHNICAL FIELDS SEARCHED (Int Cl 4)** |
| | | | C 07 C 177/00 |

The present search report has been drawn up for all claims

| Place of search | Date of completion of the search | Examiner |
|---|---|---|
| THE HAGUE | 19-04-1985 | BERTE M.J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82